# EUROPEAN PATENT SPECIFICATION

(11) **EP 4 499 610 B1**
(45) Date of publication and mention of the grant of the patent: **31.12.2025**
(21) Application number: 23719161.4
(22) Date of filing: 29.03.2023
(51) Int. Cl.: C07D 213/64, C07D 307/33

(54) **INTERMEDIATES USEFUL FOR THE PREPARATION OF AN LPXC ANALOG, AND PROCESSES FOR THEIR PREPARATION**
ZWISCHENPRODUKTE FÜR DIE HERSTELLUNG EINES LPXC-ANALOGONS UND VERFAHREN ZU IHRER HERSTELLUNG
INTERMÉDIAIRES UTILES POUR LA PRÉPARATION D'UN ANALOGUE DE LPXC, ET LEURS PROCÉDÉS DE PRÉPARATION

(30) Priority: 30.03.2022 US 202263325215 P
(43) Date of publication of application: 05.02.2025
(73) Proprietor: Zoetis Services LLC, Parsippany, NJ 07054 (US)
(72) Inventor: WALKER, Daniel, Kalamazoo, Michigan 49007 (US); SFERDEAN, Calin, Kalamazoo, Michigan 49007 (US); STUK, Timothy Lee, Kalamazoo, Michigan 49007 (US); RAMANATHAN, Shankar, Mumbai 400703 (IN)
(74) Representative: Cornwell, Matthew James
(86) International application number: PCT/US2023/016682
(87) International publication number: WO 2023/192356

(56) References cited:
- WO-A1-2011/073845
- WO-A1-2019/178305
- ZHAO, B. ET AL.: "Modulation of photochemical oxidation of thioethers to sulfoxides or sulfones using an aromatic ketone as the photocatalyst", TETRAHEDRON LETTERS, vol. 82, 31 August 2021 (2021-08-31), XP086818865, ISSN: 0040-4039, [retrieved on 20210831], DOI: 10.1016/J.TETLET.2021.153376

## Description

### FIELD OF THE INVENTION

The present invention provides a new process for preparing the LpxC hydroxamic acid inhibitor of Formula (1), (R)-4-(4-(4-(2H-1,2,3-triazol-2-yl)phenyl)-2-oxopyridin-1(2H)-yl)-N-hydroxy-2-methyl-2-(methylsulfonyl)butanamide.

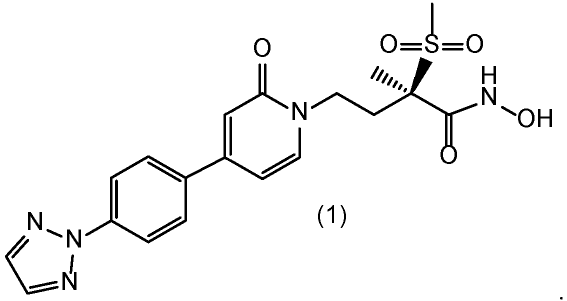

### BACKGROUND OF THE INVENTION

The LpxC inhibitor, (R)-4-(4-(4-(2H-1,2,3-triazol-2-yl)phenyl)-2-oxopyridin-1(2H)-yl)-N-hydroxy-2-methyl-2-(methylsulfonyl)-butanamide (1), is a veterinary hydroxamic acid antibiotic having biological activities against a variety of Gram-negative bacteria. Gram-negative bacteria cause a wide spectrum of diseases, including urinary tract, blood stream, gastrointestinal and airway associated infections. Gram-negative bacteria have an asymmetric bilayer composed of glycolipid lipopolysaccharides and glycerol phospholipids that make up the outer membrane of the bacteria. The Gram-negative outer membrane is an important barrier that provides protection against antibiotics and host innate immune molecules such as cationic antimicrobial peptides; and is an important construct that aids in antibacterial resistance. Recently Gram-negative bacteria with resistance to commonly used antibiotics, including quinolones, colistins (polymyxins), carbapenems, cephalosporins and other β-lactam antibiotics, have been isolated from animals with increasing frequency. Since fewer new antibiotics targeting Gram-negative bacteria are in development and organisms that resist all or most clinically useful antimicrobials are already being isolated, drug-resistant infections due to Gram-negative bacteria represent a significant health care concern for the future. Therefore, there is a need for new antibiotics against both susceptible and resistant Gram-negative bacteria.

Gram negative bacteria, such as *Escherichia coli, Salmonella and Haemophilus parasuis* have been a significant problem in the pig industry for years. Pathogens generated by these bacteria not only cause inflammation, but also cause different disease and make the pigs more susceptible to viral infection. Different strains of *E.coli* can cause piglet diarrhea and enterotoxemia, which result in decreased weight gain or even sudden death. *Salmonella enterica* can cause both pneumonia and diarrhea in the pig, as well as human food-borne gastroenteritis. Another important harmful pig bacterium, *Haemophilus parasuis,* is a pathogen that can causes fibrinous polyserositis, meningitis and arthritis, called Glässer's disease.

In recent years, many genera and species of bacteria began to show certain resistance to gram-negative antibiotics. Therefore, there is a continued need for new antibacterial agents to combat these bacterium, particularly resistant gram-negative bacteria. The LpxC inhibitor of Formula (1) has been shown to have antibacterial affect against gram-negative bacteria. Prior citations have described this molecule and the preparation thereof, *i.e.*, US Patent No. 8,664,401. However, the process described is not scalable since it only provides a yield of about 13% of a key enantiomerically pure intermediate, (R)-4-(4-chloro-2-oxopyridin-1(2H)-yl)-2-methyl-2-(methylsulfonyl)-butanoic acid (R2). Further, this process describes the use of expensive (*e.g*., Cs₂CO₃) and dangerous (*e.g.*, NaH) reagents, requires extra synthetic steps and includes an unstable intermediate, ethyl 4-bromo-2-methyl-2-(methylsulfonyl)butanoate. The process described herein provides a novel scalable process with less steps, stable intermediates, no dangerous or expensive reagents and an overall yield of 31% of the (R2) intermediate.

### SUMMARY OF THE INVENTION

In view of the number of procedural steps and use of expensive and dangerous reagents for preparing lower yields of Formula (1), as described in the art, a more robust and efficient process was developed to prepare higher and less expensive yields of the Formula (1) compound.

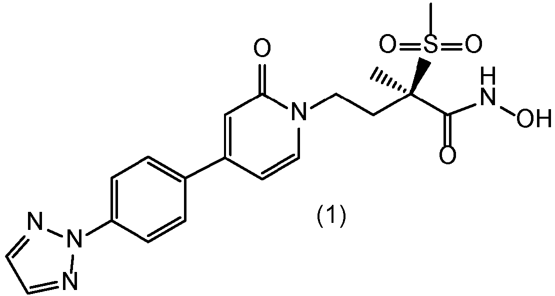

In one aspect of the invention, is the racemic intermediate compound, 3-methyl-3-(methylsulfonyl)dihydrofuran-2(3H)-one (rc1).

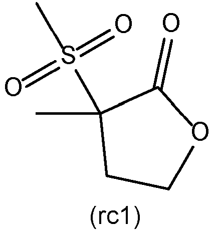

In another aspect, is a process for preparing the 3-methyl-3-(methylsulfonyl)-dihydrofuran-2(3H)-one (rc1) intermediate compound according to the following rxn-1 procedure: 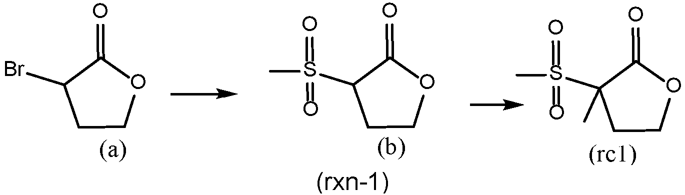 comprising:
a) reacting 3-bromotetrahydrofuran-2(3H)-one (a) with sodium methanesulfinate in the presence of solvent A, a phase transfer catalyst and optionally, a dehydrating agent, at a temperature of 40-100°C for 1-24 hours to prepare intermediate 3-(methylsulfonyl)-dihydrofuran-2(3H)-one (b);
b) cooling the mixture overnight at ambient temperature, filtering and washing the solids with solvent A;
c) reacting the solids with a base in the presence of a phase transfer catalyst, a methylating agent, solvent B, and optionally, a dehydrating agent, at a temperature of 40-100°C for a period of 4-48 hours:
d) charging the reactor with additional methylating agent and base while heating for an additional 4-hours;
e) filtering the solids while retaining the solvent filtrate;
f) washing the solids with solvent B while again retaining the solvent filtrate and then heating the combined filtrates to 65°C while distilling off all but 1.5 volumes:
g) exchanging the solvent with solvent A, cooling and seeding; and
h) cooling further to induce crystallization, filtering and washing the crystals with solvent A to afford the final product, 3-methyl-3-(methylsulfonyl)-dihydrofuran-2(3H)-one (rc1).

In another aspect, is the rxn-1 process for preparing the intermediate compound 3-methyl-3-(methylsulfonyl)-dihydrofuran-2(3H)-one (rc1) comprising:
a) reacting 3-bromotetrahydrofuran-2(3H)-one with sodium methanesulfinate in the presence of isopropanol, a phase transfer catalyst and optionally, a dehydrating agent, at a temperature of 50-80°C for 1-11 hours to prepare 3-(methylsulfonyl)dihydrofuran-2(3H)-one);
b) cooling the mixture overnight at ambient temperature, and filtering and washing the solids with isopropanol;
c) reacting the solids with potassium carbonate in the presence of a phase transfer catalyst, dimethyl sulfate, acetone, and optionally, a dehydrating agent at a temperature of 50-80°C for a period of 16-24 hours:
d) charging the reactor with additional dimethyl sulfate and potassium carbonate while heating for an additional 4-hours;
e) filtering the solids while retaining the acetone filtrate;
f) washing the solids with acetone while again retaining the acetone filtrate and heating to 65°C while distilling off all but 1.5 volumes:
g) exchanging the acetone with isopropanol, cooling to 35°C and seeding; and
h) cooling further to 25°C to induce crystallization, filtering and washing the crystals with isopropanol to afford 3-methyl-3-(methylsulfonyl)dihydrofuran-2(3H)-one.

In another aspect, is the rxn-1 process for preparing the intermediate compound 3-methyl-3-(methylsulfonyl)-dihydrofuran-2(3H)-one (rc1) comprising:
a) reacting 3-bromotetrahydrofuran-2(3H)-one with sodium methanesulfinate in the presence of isopropanol, methyltrioctylammonium chloride and optionally, the dehydrating agent magnesium sulfate, at a temperature of 80°C for about 3 hours to prepare 3-methyl-3-(methylsulfonyl)dihydrofuran-2(3H)-one;
b) cooling the mixture overnight at ambient temperature, and filtering and washing the solids with isopropanol and concentrating the solids;
c) reacting the solids with potassium carbonate in the presence of methyltrioctylammonium chloride, dimethyl sulfate and magnesium sulfate and isopropanol at a temperature of 60°C for 7 hours:
d) charging the reactor with additional dimethyl sulfate and potassium carbonate while heating for an additional 4-hours;
e) filtering the reaction mixture to remove inorganic salts;
f) washing the solids with acetone and heating to 65°C while distilling off all but1.5 volumes:
g) exchanging the acetone with isopropanol and cooling the mixture to 35°C and seeding the mixture; and
h) cooling further to 25°C to induce crystallization, filtering and washing the crystals with isopropanol to afford 3-methyl-3-(methylsulfonyl)-dihydrofuran-2(3H)-one.

In another aspect, is the rxn-1 process for preparing the intermediate compound 3-methyl-3-(methylsulfonyl)-dihydrofuran-2(3H)-one (rc1) comprising:
a) reacting 3-bromotetrahydrofuran-2(3H)-one with sodium methanesulfinate in the presence of isopropanol, methytrioctylammonium chloride and magnesium sulfate at a temperature of 80°C for 3 hours to prepare intermediate 3-(methylsulfonyl)dihydrofuran-2(3H)-one;
b) cooling the mixture overnight at ambient temperature, and isolating the solids by dissolution in isopropanol, filtering and washing the solids with isopropanol;
c) reacting the solids with potassium carbonate in the presence of methyltrioctylammonium chloride, dimethyl sulfate and magnesium sulfate in acetone at a temperature of 60°C for 7 hours:
d) charging the reactor with additional dimethyl sulfate and potassium carbonate while heating for an additional 4-hours;
e) filtering the solids while retaining the acetone filtrate;
f) washing the solids with acetone while again retaining the acetone filtrate and then heating the combined filtrates to 65°C while distilling off all but 1.5 volumes:
g) exchanging the acetone with isopropanol and cooling the mixture to about 35°C and seeding; and
h) cooling further to 25°C to induce crystallization, filtering and washing the crystals with isopropanol to afford 3-methyl-3-(methylsulfonyl)-dihydrofuran-2(3H)-one.

In another aspect, is a telescoped process (rxn-t) for preparing the intermediate compound 3-methyl-3-(methylsulfonyl)-dihydrofuran-2(3H)-one (rc1) according to the following telescoped procedure: 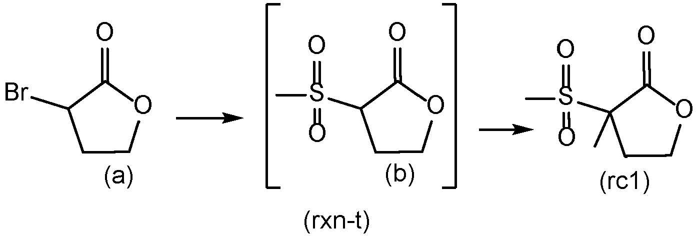 comprising:
a) reacting 3-bromodihydrofuran-2(3H)-one (a) with sodium methanesulfinate in the presence of a phase transfer catalyst, solvent B, and optionally, a dehydrating agent in a jacketed reactor for 7-11 hours at 50-80°C;
b) cooling the mixture to room temperature while stirring overnight for 12 hours; filtering the reaction mixture, retaining the filtrate, washing the solids with solvent B while retaining the filtrate;
c) reacting the combined filtrates with a methylating agent, a base and optionally, a dehydrating agent while slowly heating the reactants to 50-80°C for 15-20 hours:
d) adding more base and methylating agent to the mixture and heating for 3-6 more hours:
e) cooling the mixture to ambient temperature, filtering to remove the solids while retaining the filtrate;
f) washing the solids with solvent B, while again retaining the filtrate and heating the combined filtrates to 60-70°C; then distilling off the solvent while retaining about 1.5 volumes; and
g) exchanging solvent B with solvent A while cooling to 30-40°C and seeding; then cooling further to 20-25°C to afford 3-methyl-3-(methylsulfonyl)-dihydrofuran-2(3H)-one.

In another aspect, is the rxn-t process for preparing the intermediate compound 3-methyl-3-(methylsulfonyl)-dihydrofuran-2(3H)-one (rc1) comprising:
a) reacting 3-bromodihydrofuran-2(3H)-one with sodium methanesulfinate in the presence of a phase transfer catalyst, dehydrating agent and acetone in a jacketed reactor for 7-11 hours at 50-80°C:
b) cooling the mixture to room temperature while stirring overnight for 12 hours; filtering the reaction mixture and retaining the filtrate, washing the solids with acetone again retaining the filtrate;
c) reacting the filtrate with dimethyl sulfate, a base and dehydrating agent while slowly heating the reactants to50-80°C for 15-20 hours;
d) adding more base and methylating agent to the mixture and heating for 3-6 more hours:
e) cooling the mixture to ambient temperature, filtering to remove solids while retaining the filtrate;
f) washing the solids with acetone and again retaining the filtrate and heating the combined filtrates to 60-70°C; then distilling off the acetone while again retaining 1.5 volumes; and
g) exchanging the acetone with isopropanol while cooling to 30-40°C and seeding; then cooling further to 20-25°C to afford 3-methyl-3-(methylsulfonyl)-dihydrofuran-2(3H)-one.

In another aspect, is the rxn-t process for preparing the intermediate compound 3-methyl-3-(methylsulfonyl)-dihydrofuran-2(3H)-one (rc1) comprising:
a) reacting 3-bromodihydrofuran-2(3H)-one with sodium methanesulfinate in the presence of a methyltrioctylammonium chloride, magnesium sulfate and acetone in a jacketed reactor for 9 hours to reflux at 56°C;
b) cooling the mixture to room temperature while stirring overnight for 12 hours; filtering the reaction mixture while retaining the filtrate and washing the solids with acetone again retaining the filtrate;
c) reacting the combined filtrates with dimethyl sulfate, anhydrous potassium carbonate and anhydrous magnesium sulfate while slowly heating the reactants to reflux at 56°C for 15 hours:
d) adding more potassium carbonate and dimethyl sulfate to the mixture and heating for 4 more hours;
e) cooling the mixture to ambient temperature and filtering to remove solids while retaining the filtrate;
f) washing the solids with acetone and again retaing the filtrate and heating the combined filtrates to 65°C; then distilling off the acetone while again retaining 1.5 volumes; and
g) exchanging the acetone with isopropanol while cooling to 35°C and seeding; then cooling further to 20°C to afford 3-methyl-3-(methylsulfonyl)-dihydrofuran-2(3H)-one.

In another aspect, the reactants used for preparing the intermediate compound 3-methyl-3-(methylsulfonyl)-dihydrofuran-2(3H)-one (rc1) from 3-bromodihydrofuran-2(3H)-one include the following: the preferred base is selected from the group consisting of potassium carbonate, sodium carbonate, potassium phosphate and cesium carbonate; the preferred methylating agent is selected from the group consisting of iodomethane, bromomethane, chloromethane, dimethyl sulfate and dimethylcarbonate; the preferred phase transfer catalyst is selected from the group consisting of methyltrioctylammonium chloride, PEG 300 and PEG 400; the preferred dehydrating agent is selected from the group consisting of magnesium sulfate, sodium sulfate and calcium chloride; the preferred solvent A is selected from the group consisting of isopropanol, ethanol, methanol and acetonitrile; and the preferred solvent B is acetone or 2-butanone.

In another aspect, the reactants used for preparing the intermediate compound 3-methyl-3-(methylsulfonyl)dihydrofuran-2(3H)-one (rc1) from 3-bromodihydrofuran-2(3H)-one include the following: the more preferred base is potassium carbonate, the more preferred methylating agent is dimethyl sulfate, the more preferred phase transfer catalyst is methyltrioctylammonium chloride, the more preferred dehydrating agent is magnesium sulfate, the more preferred solvent A is isopropanol and the more preferred solvent B is acetone.

In another aspect, is a process for making the intermediate compound 3-methyl-3-(methylsulfonyl)dihydrofuran-2(3H)-one (rc1) from 3-(methylsulfonyl)dihydrofuran-2(3H)-one (b) with a dehydrating agent using potassium carbonate as the base, dimethyl sulfate as the methylating agent and acetone as solvent B. In another aspect, is a process for making the intermediate compound 3-methyl-3-(methylsulfonyl)dihydrofuran-2(3H)-one (rc1) from 3-(methylsulfonyl)-dihydrofuran-2(3H)-one (b) with a dehydrating agent using sodium carbonate as the base, iodomethane as the methylating agent and acetonitrile as solvent B. In another aspect, is a process for making the intermediate compound 3-methyl-3-(methylsulfonyl)dihydrofuran-2(3H)-one (rc1) from 3-(methylsulfonyl)dihydrofuran-2(3H)-one (b) with a dehydrating agent using potassium carbonate as the base, methyl tosylate as the methylating agent and acetone as solvent B. In another aspect, is a process for making the intermediate compound 3-methyl-3-(methylsulfonyl)dihydrofuran-2(3H)-one (rc1) from 3-(methylsulfonyl)dihydrofuran-2(3H)-one (b) with a dehydrating agent using potassium carbonate as the base, iodomethane as the methylating agent and acetone as solvent B. In another aspect, is a process for making the intermediate compound 3-methyl-3-(methylsulfonyl)dihydrofuran-2(3H)-one (rc1) from 3-(methylsulfonyl)-dihydrofuran-2(3H)-one (b) with a dehydrating agent using sodium carbonate as the base, iodomethane as the methylating agent and acetone as solvent B. In another aspect, is a process for making the intermediate compound 3-methyl-3-(methylsulfonyl)dihydrofuran-2(3H)-one (rc1) from 3-(methylsulfonyl)dihydrofuran-2(3H)-one (b) with a dehydrating agent using potassium phosphate as the base, iodomethane as the methylating agent and acetone as solvent B. In another aspect, is a process for making the intermediate compound 3-methyl-3-(methylsulfonyl)dihydrofuran-2(3H)-one (rc1) from 3-(methylsulfonyl)dihydrofuran-2(3H)-one (b) with a dehydrating agent using sodium carbonate as the base, iodomethane as the methylating agent and 2-butanone as solvent B.

In another aspect, is a process for making the intermediate compound 3-methyl-3-(methylsulfonyl)dihydrofuran-2(3H)-one (rc1) from 3-(methylsulfonyl)-dihydrofuran-2(3H)-one (b) without a dehydrating agent using potassium carbonate as the base, dimethyl sulfate as the methylating agent and acetone as solvent B. In another aspect, is a process for making the intermediate compound 3-methyl-3-(methylsulfonyl)-dihydrofuran-2(3H)-one (rc1) from 3-(methylsulfonyl)-dihydrofuran-2(3H)-one (b) without a dehydrating agent using sodium carbonate as the base, iodomethane as the methylating agent and acetonitrile as solvent B. In another aspect, is a process for making the intermediate compound 3-methyl-3-(methylsulfonyl)-dihydrofuran-2(3H)-one (rc1) from 3-(methylsulfonyl)dihydrofuran-2(3H)-one (b) without a dehydrating agent using potassium carbonate as the base, methyl tosylate as the methylating agent and acetone as solvent B. In another aspect, is a process for making the intermediate compound 3-methyl-3-(methylsulfonyl)dihydrofuran-2(3H)-one (rc1) from 3-(methylsulfonyl)dihydrofuran-2(3H)-one (b) without a dehydrating agent using potassium carbonate as the base, iodomethane as the methylating agent and acetone as solvent B. In another aspect, is a process for making the intermediate compound 3-methyl-3-(methylsulfonyl)dihydrofuran-2(3H)-one (rc1) from 3-(methylsulfonyl)-dihydrofuran-2(3H)-one (b) without a dehydrating agent using sodium carbonate as the base, iodomethane as the methylating agent and acetone as solvent B. In another aspect, is a process for making the intermediate compound 3-methyl-3-(methylsulfonyl)-dihydrofuran-2(3H)-one (rc1) from 3-(methylsulfonyl)dihydrofuran-2(3H)-one (b) without a dehydrating agent using potassium phosphate as the base, iodomethane as the methylating agent and acetone as solvent B. In another aspect, is a process for making the intermediate compound 3-methyl-3-(methylsulfonyl)dihydrofuran-2(3H)-one (rc1) from 3-(methylsulfonyl)dihydrofuran-2(3H)-one (b) without a dehydrating agent using sodium carbonate as the base, iodomethane as the methylating agent and 2-butanone as solvent B.

In another aspect of the invention, is the enantiomerically pure intermediate compound (R)-3-methyl-3-(methylsulfonyl)dihydrofuran-2(3H)-one (R1).

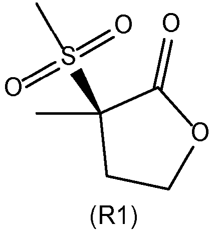

In another aspect, is a process for preparing the enantiomerically pure intermediate compound (R1) from the racemic (rc1) substrate according to the following procedure using chiral chromatography.

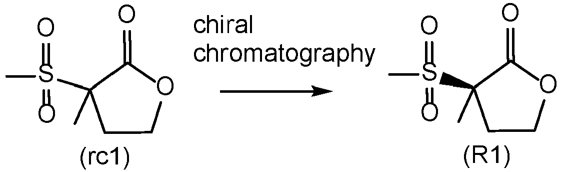

In another aspect, chiral separation of the enantiomerically pure intermediate compound (R1) from the racemic substrate (rc1) can be accomplished with chiral chromatography. In another aspect, the chiral chromatography uses a Chiralpak^{®} or Chiracel^{®} column. In another aspect, the Chiralpak^{®} chromatography column is an AD, AS, AY, AZ, IA, IB, IC, ID, IE, IF or IG column. In another aspect, the Chiralpak^{®} chromatography column is an AD Chiralpak^{®} column. In another aspect, the enantiomerically pure intermediate compound (R1) can be separated from the racemic (rc1) substrate with a Chiralpak^{®} AD chromatography column. In another aspect, the Chiracel^{®} chromatography column is an OD or OJ Chiracel^{®} column. In another aspect, the enantiomerically pure intermediate compound (R1) can be separated from the racemic (rc1) substrate with a Chiralcel^{®} OD or OJ chromatography column. In another aspect, the enantiomerically pure intermediate compound (R1) can be separated from the racemic (rc1) substrate with a Chiralcel^{®} OD chromatography column. In another aspect, the enantiomerically pure intermediate compound (R1) can be separated from the racemic (rc1) substrate with a Chiralcel^{®} OJ chromatography column.

In another aspect, is a process for preparing the intermediate compound (rc2) from (rc1) by reacting (rc1) with the pyridinone in the presence of a base and a solvent to prepare (rc2) according to the following procedure:

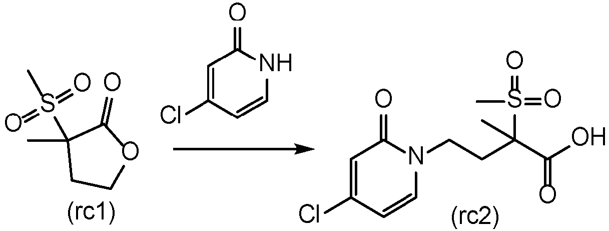

In another aspect, is a process for preparing the intermediate compound 4-(4-chloro-2-oxopyridin-1(2H)-yl)-2-methyl-2-(methylsulfonyl)butanoic acid (rc2) from 3-methyl-3-(methylsulfonyl)dihydrofuran-2(3H)-one (rc1) comprising:
a) dissolving potassium tert-butoxide in anhydrous DMSO and then adding 4-chloropyridin-2(1H)-one while maintaining the temperture between 18-27°C;
b) adding in a mixture of (rc1) and benzyltriethylammonium bromide and heating to 85°C for 2 hours;
c) aging the reactants for 17-20 hours at 85°C;
d) adding isopropyl acetate and stirring for 1 hour at 60°C;
e) cooling to 20°C over an hour and aged for an hour; filtering the solids and washing with isopropyl acetate and drying overnight at 50°C to yield the potassium salt of (rc2);
f) dissolving the dried solids in water and treating with methanol and methyl tetrahydrofuran and charging with concentrated HCl;
g) stirring the slurry at room temperature for 2 hours and cooling to 0-5°C while stirring for an additional hour:
h) filtering the slurry and washing wth water:MeOH (8:2) and then drying in vacuo under nitrogen to afford (rc2).

In another aspect, is a process for preparing the intermediate compound 4-(4-chloro-2-oxopyridin-1(2H)-yl)-2-methyl-2-(methylsulfonyl)butanoic acid (rc2) from 3-methyl-3-(methylsulfonyl)dihydrofuran-2(3H)-one (rc1) comprising:
a) charging a reactor under nitrogen with PEG, 2-methyl-2-butanol and 4-chloro-2-hydroxypyridine;
b) adjusting the reactor temperature to 18-25°C and adding potassium tert-butoxide while stirring;
c) aging the reaction mixture for30-60 minutes at 40-50°C;
d) adding (rc1) to the reactor and heating to 100°C over 15-30 minutes;
e) maintaining the temperature at 95-100°C for 12-24 hours
f) cooling the reactants to 50-60°C, diluting with water and adjusting the pH with HCl to 1-2;
g) cooling the reactants to 0-5°C over 1-3 hours, aging for 1-2 hours and filtering the resulting solids; and
h) washing the solids with water and drying to afford (rc2).

In another aspect, is a process for preparing intermediate compound 4-(4-chloro-2-oxopyridin-1(2H)-yl)-2-methyl-2-(methylsulfonyl)butanoic acid (rc2) from 3-methyl-3-(methylsulfonyl)dihydrofuran-2(3H)-one (rc1) comprising:
a) charging a reactor under nitrogen with PEG 400, 2-methyl-2-butanol and 4-chloro-2-hydroxypyridine;
b) adjusting the reactor temperature to 20°C and adding potassium tert-butoxide portion-wise while stirring;
c) aging the reaction mixture for 30-45 minutes at 40-45°C;
d) adding (rc1) to the reactor and heating to 100°C over 20 minutes;
e) maintaining the temperature at 95-100°C for 16-20 hours
f) cooling the reactants to 50-60°C, diluting with water and adjusting the pH with HCl to 1-2;
g) cooling the reactants to 0°C over 2 hours, aging for 1 hour and filtering the resulting solids; and
h) washing the solids with deionized water and drying at 70°C to afford (rc2).

Also provided is the intermediate compound 4-(4-chloro-2-oxopyridin-1(2H)-yl)-2-methyl-2-(methylsulfonyl)butanoic acid, (rc2).

In another aspect, is a process for preparing the intermediate compound (R)-4-(4-chloro-2-oxopyridin-1(2H)-yl)-2-methyl-2-(methylsulfonyl)butanoic acid (R2) from the enantiomerically pure (R)-3-methyl-3-(methylsulfonyl)dihydrofuran-2(3H)-one (R1) according to the following procedure 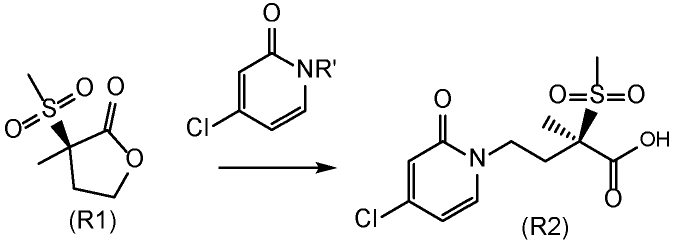 by reacting (R1) with the pyridinone in a solvent to prepare (R2); wherein R' is H or K.

In another aspect, is a process for preparing the intermediate compound (R)-4-(4-chloro-2-oxopyridin-1(2H)-yl)-2-methyl-2-(methylsulfonyl)butanoic (R2) acid from (R)-3-methyl-3-(methylsulfonyl)dihydrofuran-2(3H)-one (R1) comprising:
a) dissolving potassium tert-butoxide in anhydrous DMSO or sulfolane and then adding 4-chloropyridin-2(1H)-one while maintaining the temperture between 18-27°C;
b) adding in a mixture of (R)-3-methyl-3-(methylsulfonyl)dihydrofuran-2(3H)-one (R1) and benzyltriethylammonium bromide and heating to 85°C for 2 hours;
c) aging the reactants for 17-20 hours at 85°C;
d) adding isopropyl acetate and stirring for 1 hour at 60°C;
e) cooling to 20°C over an hour and aged for an hour; filtering the solids and washing with isopropyl acetate and drying overnight at 50°C to yield the potassium salt of (R)-4-(4-chloro-2-oxopyridin-1(2H)-yl)-2-methyl-2-(methylsulfonyl)butanoic acid;
f) dissolving the dried solids in water and treating with methanol and methyl tetrahydrofuran and charging with concentrated HCl;
g) stirring the slurry at room temperature for 2 hours and cooling to 0-5°C while stirring for an additional hour; and
h) filtering the slurry and washing wth water:MeOH (8:2) and then drying in vacuo under nitrogen to afford (R)-4-(4-chloro-2-oxopyridin-1(2H)-yl)-2-methyl-2-(methylsulfonyl)butanoic acid.

Also provided is the intermediate compound (R)-4-(4-chloro-2-oxopyridin-1(2H)-yl)-2-methyl-2-(methylsulfonyl)butanoic acid, (R2).

In another aspect, is the (S)-enantiomerically pure intermediate compound (S1), 3-methyl-3-(methylsulfonyl)dihydrofuran-2(3H)-one

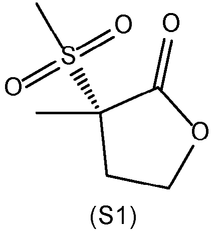

### DETAILED DESCRIPTION

### Description of Figure(s)

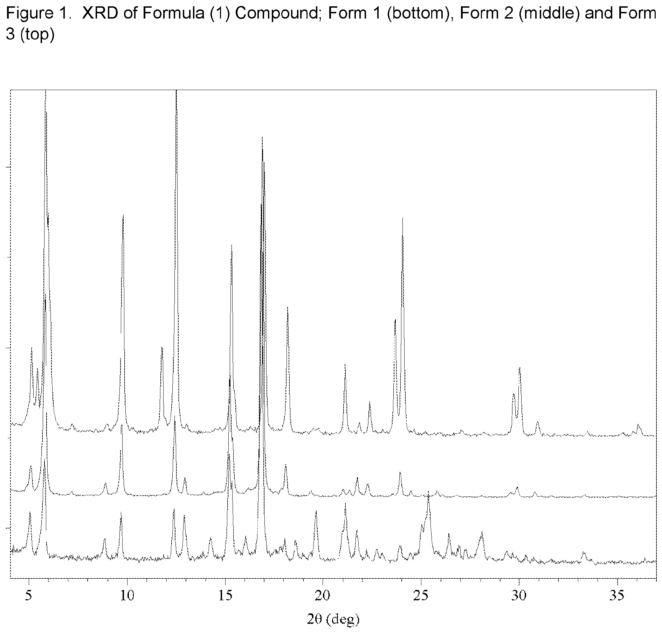

Figure 1. Powder X-Ray Diffractograms of Form 1, Form 2 and Form 3 of the Formula (1) compound, (R)-4-(4-(4-(2H-1,2,3-triazol-2-yl)phenyl)-2-oxopyridin-1(2H)-yl)-N-hydroxy-2-methyl-2-(methylsulfonyl)-butanamide.

For purposes of the present invention, as described and claimed herein, the following terms and phrases are defined as follows:
"About" when used in connection with a measurable numerical variable, refers to the indicated value of the variable and to all values of the variable that are within the experimental error of the indicated value (e.g., within the 95% confidence interval for the mean) or within 10 percent of the indicated value, whichever is greater. The term can be readily removed as a descriptor.
"Animal(s)", as used herein, unless otherwise indicated, refers to an individual animal that is a mammal. Specifically, mammal refers to a vertebrate animal that is non-human, which are members of the taxonomic class Mammalia. Examples of non-human mammals include companion animals (i.e., feline, canine and equine) and livestock (i.e., swine, bovine, ovine and caprine). The preferred livestock is swine and bovine; the more preferred is swine.
"Pharmaceutically acceptable" as used herein, unless otherwise indicated, indicates that the substance or composition must be compatible chemically and/or toxicologically, with the other ingredients comprising a composition (formulation) and/or the animal being treated therewith. The use of the phrase "and pharmaceutically acceptable salt(s)" can be interchanged with "or a pharmaceutically acceptable salt(s)".
"Seeding", as used herein, unless otherwise indicated, indicates that a small sample of crystal or polycrystalline material (e.g., reference standard) for the final intermediate or product to be afforded from the reaction is added to the supersaturated reaction mixture to promote growth of the crystal wanted thereby potentiating intermolecular forces between the separate molecules in solution to form the basis for a crystal lattice and eliminating the need for random molecular collision or interaction.

As described herein, the following intermediates (compounds) and respective names are synonymous:
(a) = 3-bromodihydrofuran-2(3H)-one;
(b) = 3-(methylsulfonyl)dihydrofuran-2(3H)-one;
(rc1) = 3-methyl-3-(methylsulfonyl)dihydrofuran-2(3H)-one (racemic);
(rc2) = 4-(4-chloro-2-oxopyridin-1(2H)-yl)-2-methyl-2-(methylsulfonyl)butanoic acid (racemic);
(R1) = (R)-3-methyl-3-(methylsulfonyl)dihydrofuran-2(3H)-one;
(R2) = (R)-4-(4-chloro-2-oxopyridin-1(2H)-yl)-2-methyl-2-(methylsulfonyl)-butanoic acid; and
(S1) = (S)-3-methyl-3-(methylsulfonyl)dihydrofuran-2(3H)-one.

The LpxC inhibitor, Formula (1), (R)-4-(4-(4-(2H-1,2,3-triazol-2-yl)phenyl)-2-oxopyridin-1(2H)-yl)-N-hydroxy-2-methyl-2-(methylsulfonyl)-butanamide, is a veterinary hydroxamic acid antibiotic having biological activity against a variety of Gram-negative bacteria. The compound can be formulated as a free base with at least one other pharmaceutically acceptable excipient or as a salt with at least one other pharmaceutically acceptable excipient. For the purpose of this process, the hydroxamic acid Formula (1) compound can be formulated for use to treat Gram-negative bacteria in livestock, *i.e*., bovine and swine; and preferably swine.

The racemic intermediate compound (rc1) has a chiral center and thus includes a racemic mixture of two enantiomers. Each enantiomer has identical chemical and physical properties except for their ability to rotate plane-polarized light (+/-) by equal amounts but in opposite directions. Enantiomers are also called optical isomers. A mixture of equal parts of an optically active isomer and its enantiomer has a zero-net rotation of plane-polarized light because the positive rotation of each (+) form is exactly counteracted by the negative rotation of each (-) form. Separation of the (R1) enantiomer from the racemic mixture can be accomplished by standard chromatographic methods on chiral adsorbents. Alternatively, the racemate can be coupled with reactant (c) to prepare (rc2) which can be resolved to (R2) through classical resolution techniques by reacting (rc2) with optically pure (S)-(+)-α-methylbenzylamine.

### Pharmaceutical Salts

The Formula (1) compound may be used in its native form (base) or as a salt. The Formula (1) compound has a basic functional group and can form addition salts with acids. Such salts are included within the scope of the present invention to the extent that they are acceptable for veterinary use. Suitable acid addition salts are formed from acids which form non-toxic salts. Examples include the acetate, adipate, aspartate, benzoate, besylate, bicarbonate/carbonate, bisulphate/sulphate, borate, camsylate, citrate, cyclamate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulphate, naphthylate, 2-napsylate, icotinate, nitrate, orotate, oxalate, palmitate, pamoate, phosphate/hydrogen phosphate/dihydrogen phosphate, pyroglutamate, saccharate, stearate, succinate, tannate, tartrate, tosylate, trifluoroacetate and xinafoate salts.

### Composition/Formulation

Pharmaceutical compositions comprising the Formula (1) compound may be manufactured by processes well known in the art, *e.g.,* by means of conventional mixing, dissolving, granulation, levigating, emulsifying, encapsulating, entrapping, lyophilizing processes or spray drying.

Pharmaceutical compositions for use in accordance with the Formula (1) compound may be formulated in conventional manner using one or more pharmaceutically acceptable excipients, which facilitate processing of the active compound into preparations, which can be used for pharmaceutical administration to the animal in need thereof. Proper formulation is dependent upon the route of administration chosen. Pharmaceutically acceptable excipients are generally known to those skilled in the art and are thus included in the instant invention. Such excipients are generally described, for example, in "Remington's Pharmaceutical Sciences", Mack Pub. Co., New Jersey (1991). In one aspect, the composition comprising the Formula (1) compound is prepared as an oral, parenteral (intravenous, intramuscular and sub-cutaneous) or topical composition. The preferred route of administration is oral administration, and particularly for administration in drinking water. The preferred animal to receive the composition is livestock, preferably bovine and swine. The most preferred is swine. The oral composition can be formulated with other excipients. Other commonly used excipients to prepare a pharmaceutical composition can include, for example, any one or more of the following: anti-oxidant (*e.g*., BHT, BHA, vitamin E, sodium metabisulfite, propyl gallate and the like); preservative (*e.g.,* benzyl alcohol, citric acid, alkyl-paraben, phenol, meta-cresol and the like); antifoaming agent (*e.g.,* cetostearyl alcohol, castor oil, stearates, polydimethylsiloxanes, glycols and the like); surfactant (*e.g*., polysorbates, sodium lauryl sulfate, lauryl dimethyl amine oxide, polyethoxylated alcohols, polyoxyethylene sorbitan, octoxynol, polyoxyl 10 lauryl ether, polyoxyl castor oil, cyclodextrins, lecithin and the like); and hydrotropic agents (*e.g*., nicotinamide, benzamide, N,N-diethylnicotinamide, nicotinic acid, sodium benzoate, urea, potassium or sodium salycilate and the like) which can increase the water solubility of the Formula (1) compound. The Formula (1) compound can be prepared in a concentrated amount, for example 10mg/mL, 25mg/mL, 50mg/mL, 100mg/mL, 150mg/mL, 200mg/mL, 250mg/mL and higher. The Formula (1) compound can be administered to the animal in need thereof at doses ranging from about 0.25 to about 2.5mg/kg; or a dosing amount of about 0.5 to about 2mg/kg; or a dosing amount of about 0.75 to about 1.5mg/kg; or a dosing amount of about 1 to about 1.25mg/kg. The Formula (1) compound can be administered to the animal in need thereof at doses ranging from 0.25 to 2.5mg/kg; or a dosing amount of 0.5 to 2mg/kg; or a dosing amount of 0.75 to 1.5mg/kg; or a dosing amount of 1 to 1.25mg/kg.

### Veterinary Uses

The Formula (1) compound may be used for the treatment or prevention of infectious disorders, especially those caused by susceptible and multi-drug resistant Gram-negative bacteria in animals. Non-limiting examples of such Gram-negative bacteria include *Acinetobacter baumannii, Acinetobacter spp., Achromobacter* spp., *Aeromonas* spp., *Actinobacillus pleuropneumoniae, Bacteroides fragilis, Bordetella* spp., *Borrelia* spp., *Brucella* spp., *Campylobacter* spp., *Citrobacter diversus* (*koseri*)*, Citrobacter freundii, Enterobacter aerogenes, Enterobacter cloacae, Escherichia coli, Francisella tularensis, Fusobacterium* spp., *Haemophilus parasuis, Helicobacter pylori, Klebsiella oxytoca, Klebsiella pneumoniae, Lawsonia intracellularis, Legionella pneumophila, Moraxella catarrhalis* (*ß*-lactamase positive and negative), *Morganella morganii, Neisseria gonorrhoeae, Neisseria meningitidis, Proteus vulgaris, Porphyromonas* spp., *Prevotella* spp., *Mannheimia haemolyticus, Pasteurella* spp., *Proteus mirabilis, Providencia* spp., *Pseudomonas aeruginosa, Pseudomonas* spp., *Salmonella* spp., *(S. enterica) Shigella* spp., *Serratia marcescens, Treponema* spp., *Burkholderia cepacia, Vibrio* spp., *Yersinia* spp., and *Stenotrophomonas mulophilia.*

Examples of infections that may be treated with the Formula (1) compound include nosocomial pneumonia, urinary tract infections, systemic infections (bacteremia and sepsis), skin and soft tissue infections, lung infections, surgical infections, intra-abdominal infections and the like. The preferred use of the Formula (1) compound is to treat animals against Gram-negative bacteria, preferably livestock (*e.g*., bovine and swine) and more preferably swine.

### Examples

The following acronyms described herein include: ethanol (EtOH); isopropanol (IPA); methyl (Me); ethyl (Et); ethyl acetate (EtOAc), potassium acetate (KOAc), sodium hydride (NaH); methanol (MeOH); sodium carbonate (Na₂CO₃); polyethylene glycol (PEG); hydrochloric acid (HCl); acetonitrile (MeCN, CH₃CN, Acn); potassium carbonate (K₂CO₃); oxalyl chloride (CsO₂Cl₂); potassium phosphate (K₃PO₄), methyl triflate (MeOTf); methyl tosylate (MeOTs); dimethyl sulfate ((CH₃)₂SO₄); cesium carbonate (Cs₂CO₃); trifluoroacetic acid (TFA); tetrahydrofuran (THF); calcium oxide (CaO); potassium hydroxide (KOH); sodium hydroxide (NaOH); sodium bromide (NaBr); dichloromethane (DCM); tetrahydropyran (THP); potassium acetate (KOAc); potassium tert-butoxide (KOtBu); sodium borohydride (NaBH₄); hydrobromic acid (HBr); aluminum oxide (Al₂O₃); 2-dicyclohexylphosphino-2',4',6'-triisopropyl-1,1'-biphenyl (XPhos); magnesium sulfate (MgSO₄); sodium sulfate (Na₂SO₄); calcium chloride (CaCl₂); iodomethane (Mel); dimethyl sulfoxide (DMSO); bromomethane (MeBr); chloromethane (MeCl); methyllithium (MeLi); butylated hydroxytoluene (BHT); butylated hydroxyanisole (BHA); dibromoethane (DBE); lithium hydroxide (LiOH); chloro(2-dicyclohexylphosphino-2',4',6'-triisopropyl-1,1'-biphenyl)[2-(2'-amino-1,1'-biphenyl)]palladium(II), 2^{nd} generation (XPhos PD G2); tetrahydroxydiboron (B₂(OH)₄); sodium methanesulfinate (MeSO₂Na); aqueous (aq); equivalent (eq); not more than (NMT); not less than (NLT); catalyst (cat); limit of detection (LOD); volume (vol); minute(s) (min); melting point (mp); and room temperature (RT).

Schemes 1-3 outline the general procedures for the preparation and isolation (or not (*i.e*., telescoped)) of the intermediate compounds (b), (rc1), (R1), (rc2), (R2) and the Formula (1) compound. It is to be understood, however, that the invention, as fully described herein and as recited in the claims, is not intended to be limited by the details of the following schemes or modes of preparation. The starting materials and various reactants can be obtained from commercial sources, or are readily prepared from commercially available organic compounds, using well-known methods to one skilled in the art.

Alternatively, the intermediate compound (R2) and the Formula (1) compound can be prepared by methods described in US Patent No. 8,664,401 and PCT publication WO2019178305. The method for preparing the intermediate compound (R2) using the processes described herein has numerous advantages over the prior methods, for example:
1) Step count to (R2) is 4 or 6 for the chiral and classical resolution routes, respectively, versus 7 steps;
2) Elimination of the unstable intermediate, ethyl 4-bromo-2-methyl-2-(methylsulfonyl)butanoate with the novel lactone intermediate (rc1);
3) Increased yield more than 2-fold from 13% to 31% (max of 50%);
4) Increased scalability by eliminating expensive (Cs₂CO₃) and dangerous (NaH) reagents; and
5) potential for a telescoped (non-isolated) reaction step(s).

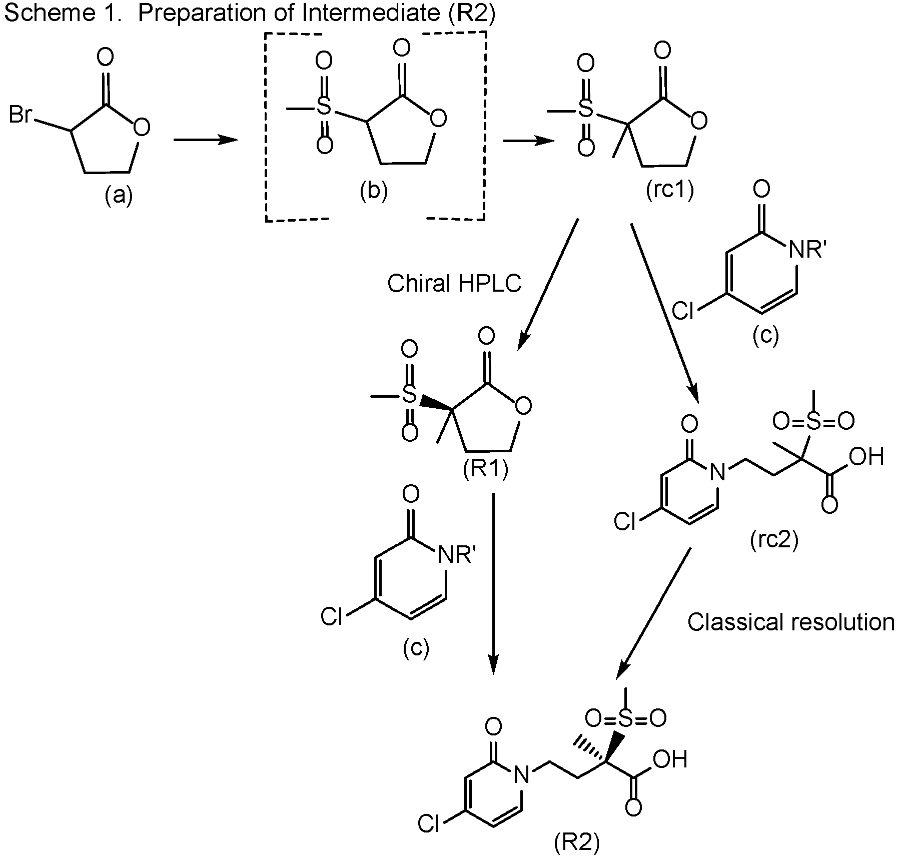

As shown in Scheme 1, treatment of commercial α-bromo-γ-butyrolactone (a) with sodium methanesulfinate afforded sulfone (b), which, depending on the solvent used, can be either isolated or [telescoped] into the next step. Exposure of (b) to a base (*e.g*., sodium carbonate, potassium carbonate and the like) in the presence of a methylating agent (*e.g.*, methyl iodide, dimethyl sulfate, and the like) gave rise to (rc1). The carboxylic acid (R2) was realized by two different routes, one which relies on classical resolution and the other relying on chiral chromatographic resolution. Alkylation of lactone (rc1) with pyridinone (c) [wherein R' is H or K] in the presence of base (*e.g*., potassium tert-butoxide, sodium *tert*-amylate, and the like) afforded (rc2). Classical resolution of (rc2) was accomplished using a chiral amine (*e.g.*, α-methylbenzylamine, ephedrine, phenyl glycinol, and the like), affording (R2) as a single enantiomer.

Alternatively, chromatographic resolution of (rc1) afforded (R1) as a single enantiomer. Chromatography was carried out according to methods known in the art (Geoffrey B. Cox Preparative Enantioselective Chromatography, Blackwell Publishing, Ltd: Ames, IA; 2005) using a chiral stationary phase (e.g., ChiralPak AD, Chiralcel OJ, and the like) and a mobile phase consisting of an appropriate HPLC solvent (e.g., methanol, ethanol, isopropanol, heptane, methyl tert-butyl ether, and the like). Alkylation of lactone (R1) with pyridinone (c) in the presence of a base (e.g., potassium tert-butoxide, sodium tert-amylate and the like) afforded (R2).

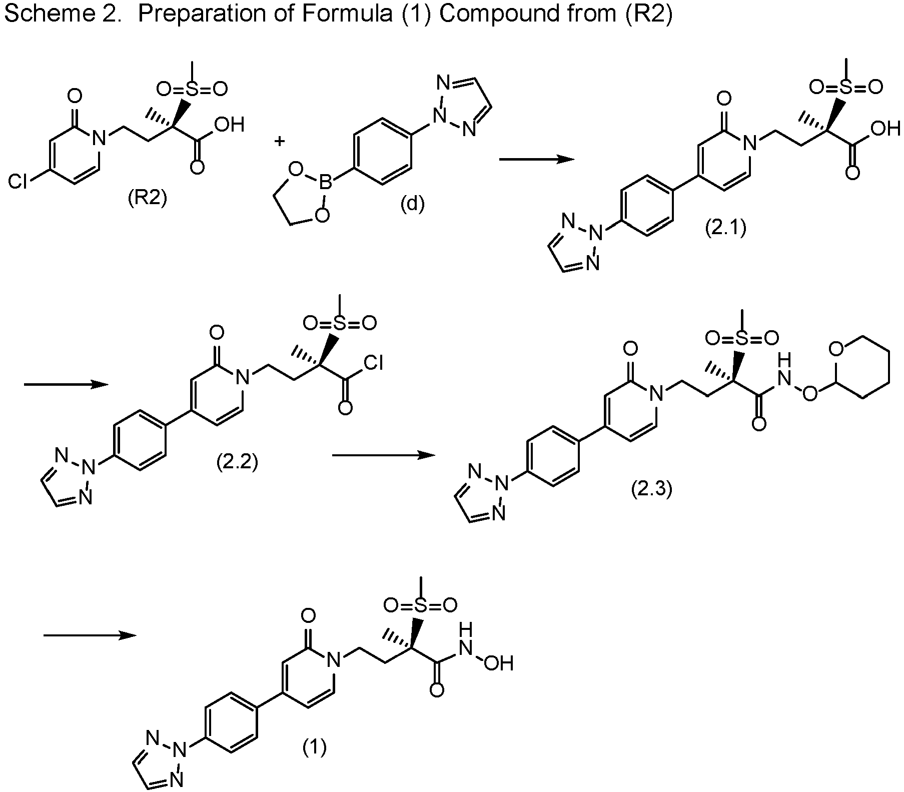

As described in Scheme 2, analog (2.1) was obtained via a palladium catalyzed Suzuki cross coupling of pyridinone chloride (R2) with borolane (d). Alternatively, 2-(4-(4,4,5,5-tetramethyl-1,3,2-dioxaborolan-2-yl)phenyl)-2H-1,2,3-triazole can be used in lieu of (d). Carboxylic acid (2.1) was converted to the corresponding acid chloride (2.2) using typical reagents known in the art, such as oxalyl chloride, thionyl chloride, and the like. Subjection of the acid chloride (2.2) to tetrahydropyranyl-protected hydroxyl amine afforded intermediate (2.3). Cleavage of the tetrahydropyranyl group using typical reagents to those known in the art, for example, an aqueous acid (e.g., hydrochloric, sulfuric, maleic, phosphoric, oxalic, citric, tartaric, methane sulfonic acid, p-toluenesulfonic acid, pyridinium p-toluene sulfonate and the like), afforded the Formula (1) compound. A preferred cleavage reagent is sulfuric acid or maleic acid. Additional chemical descriptions for this process can be found in US Patent No. 8,664,401 and PCT publication WO2019/178305.

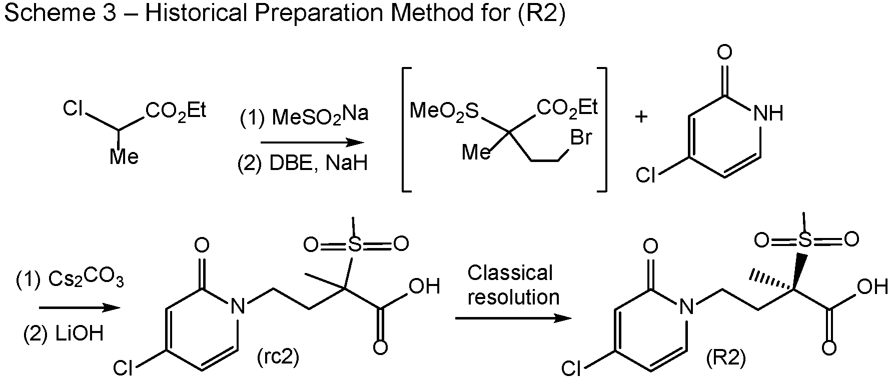

According to Scheme 3, treatment of ethyl 2-chloropropionate with sodium methanesulfinate, followed by subsequent exposure to sodium hydride and dibromoethane afforded the bromide. Alkylation of chloropyridinone, prepared according to the literature procedure (Honraedt and Gallagher SynLett, 2016, 27, 67), with the bromide in the presence of cesium carbonate followed by subsequent saponification of the ester with lithium hydroxide gave rise to (rc2). Classical resolution of (rc2) using (-)-ephedrine was conducted according to known procedures known (*i.e.,* Brown et.al., J. Med. Chem. 2021, 55, 914) to afford (R2) as a single enantiomer.

As described herein, the enantiomerically pure enantiomer, (R1), can be resolved from the racemic mixture (rc1) or substrate by chiral chromatography techniques. For example, Chiralpak^{®} AD, AS, AY, AZ, IA, IB, IC, ID, IE, IF, IG column or Chiralcel^{®} OD or OJ column; both with a solvent mobile phase selected from any one of methanol, ethanol, isopropanol, acetonitrile, acetone, ethyl acetate, THF, CH₂Cl₂, MTBE and heptane, or mixtures thereof. The preferred HPLC solvent is selected from methanol, ethanol and heptane. The most preferred HPLC solvent is methanol.

The preferred column for separating the (R1) enantiomer from the racemate, (rc1), is the Chiralpak^{®} AD. The column is packed with coated polysaccharide-based chiral stationary phases that are manufactured by physically coating the polysaccharide derivatives onto silica matrices. Chromatographic separation was conducted at a temperature of about 20-40°C, preferably at about 20-35°C and more preferably at about 25°C.

Non-limiting bases that can be used in the preparation of (R2) from intermediate (b) including, for example, Na₂CO₃, K₂CO₃, Cs₂CO₃, K₃PO₄, alkali metal tert-butoxides (*e.g.*, potassium-, lithium-, sodium- and rubidium-tert-butoxide), sodium tert-amylate, and the like. The preferred base is K₂CO₃, Na₂CO₃ or K₃PO₄; and the more preferred base is K₂CO₃.

Methylation denotes the addition of a methyl group on a substrate, *i.e.,* a form of alkylation; the replacement of a H atom with a methyl group. Non-exclusive methylating agents include, for example: iodomethane (CH₃I), bromomethane (CH₃Br), chloromethane (CH₃Cl), methyllithium (CH₃Li), diazomethane, dimethyl carbonate, dimethyl dicarbonate, dimethyl sulfate, 1,2-dimethylhydrazine, dimethyl zinc, methyl fluorosulfinate, methyl cobalamin, methyl magnesium chloride, trimethylphosphate, methyl tosylate, methylmethane sulfonate, N-methyl-N'-nitrosoguanidine and the like. The preferred methylating agent is selected from dimethyl sulfate, bromomethane and iodomethane. The most preferred methylating agent is dimethyl sulfate.

Non-exclusive examples of organic solvents (solvents), include, for example, alcohols (ROH) wherein R is a linear, branched or cyclic alkyl having from 1 to 8 carbon atoms (*e.g.,* methanol, ethanol, propanol, isopropanol, n-propanol, 2-butanol, n-butanol, t-butanol, isobutanol, cylopentanol, phenol, benzyl alcohol, and the like); nitrenes (*e.g.,* acetonitrile, propriononitrile, butyronitrile, isobutyronitrile, 2-methylbutanenitrile, pentanenitrile and the like); ketones (*e.g.,* acetone, butanone, pentanone, 2-methylpentanone and the like); ethers (*e.g.,* methoxymethane, methoxyethane, ethoxyethane, ethyl ether, diethyl ether, tetrahydrofuran, methoxybenzene, and the like); esters (*e.g.,ethyl* acetate, methyl formate, methyl acetate, ethyl proprionate, isopropyl butyrate, ethyl benzoate, and the like); dimethyl sulfoxide (DMSO), sulfolane, 3-methyl sulfolane, dimethylformamide, dimethylacetamide, ethylene glycol, pyridine, toluene, dioxane and the like. Preferred solvents include isopropanol, acetone, acetonitrile, 2-butanol, sulfolane, t-butanol and DMSO. The more preferred solvents are isopropanol, acetone, 2-butanol, acetonitrile and sulfolane. A preferred solvent A is isopropanol, ethanol, methanol and acetonitrile. The more preferred solvent A is isopropanol. A preferred solvent B is acetone or 2-butanone. The more preferred solvent B is acetone.

Phase transfer catalysts are used to enable the reaction in a heterogenous system between organic compounds soluble in organic solvents (solvents) and compounds soluble in water, such as inorganic salts. The phase transfer catalyst is soluble in both solvents, and it carries anions of inorganic salts into organic solvents (solvents) and returns them into the water phase. Reactions usually progress under mild conditions with easy work-up procedures. For this reason, they are also used industrially. Typical phase-transfer catalysts Include polyethylene glycol (PEG), quaternary ammonium salts, crown ethers and phosphonium compounds. Some non-exclusive examples of phase transfer catalysts include, for example, PEG 400, PEG 300, benzyltriethylammonium chloride, methyltricaprylammonium chloride, methyltributylammonium chloride, tetrabutylammonium thiocyante, tetrabutylphosphonium bromide, methyltrioctylammonium chloride (Aliquat^{®}336), tetrabutylammonium bromide, methyltrioctylammonium bromide and the like. The preferred phase transfer catalysts are PEG 400 and Aliquat^{®}336. Aliquat^{®}336 is a methyltrialkyl-ammonium salt with C8 and C10 alkyl groups [(C(8 and 10)3NMe]. The Aliquat trademark is a registered mark of Henkel Corporation, USA.

Dehydrating agents can also be used in the reactions described herein to dry and remove water. Non-exclusive examples of dehydrating agents include: MgSO₄, Na₂SO₄, CaCL₂, zinc chloride, NaBH₄, Al₂O₃, calcium oxide (CaO), silica gel and sieves. The preferred dehydrating agent is MgSO₄.

Use of a resolving agent is a common method of resolution to convert the racemic mixture of a compound into a mixture of diasteromers by reacting it with a single enantiomer of a chiral agent, known as the resolving agent, separate the components of the mixture of diastereomers, and chemically convert each diastereomer into the corresponding enantiomer. Non-exclusive resolving agents for classical resolution of (rc2) to (R2) include, for example, (S)-methylbenzylamine, phenyl glycinol, ephedrine, cinconidine, 2-amino-1,2-diphenylethanol, quinine, quinidine and the like. The preferred resolving agents are (S)-methylbenzylamine and phenyl glycinol. The most preferred resolving agent is (S)-methylbenzylamine.

### EXAMPLES

Proton (¹H) nuclear magnetic resonance (NMR) spectra were collected on a Bruker Avance III 600 MHz Spectrometer or a Bruker Avance III HD 400 MHz, each with a 5mm Broadband probe at ambient temperature. Chemical shifts are reported in parts per million (δ) relative to tetramethylsilane (δ=0.0) and are referenced to residual solvent resonances (CDCl₃ δ = 7.27, and DMSO-*d₆* δ = 2.50).

Lactone Opening Reactions were monitored by the following Reverse Phase HPLC methods:
A) Reverse phase HPLC conditions: Agilent 1100 HPLC, 1mL/min, 40°C column temp, 230nm UV detection, Mobile Phase: solvent x (0.1% aqueous TFA) and solvent y (Acn/MeOH (1:1 v/v)). Method: Gradient: Solvent x 5% to 50% over 50 minutes and then 50% to 100% over 5 minutes.
   Column: Supelco Ascentis Express C18 150 x 4.6 2.7 µm, Injection volume 5µL. Samples were prepared by diluting 10µL of reaction mixture/mother liquor with 1mL of water/acetonitrile containing citric acid or 0.5mg/mL in solvent y for solid samples. Retention times: chloropyridone (4.5 minutes), O-alkylated pyridine (12.7 minutes), N-alkylated pyridine (R2; 7.7 minutes), compound 2.1 (13.6 minutes), compound 2.3 (18.4 minutes) and the Formula (1) compound (11.8 minutes).
B) Reverse phase Chiral HPLC conditions: Agilent 1100 HPLC, 0.7mL/min, 15°C column temp, 218 and 230 nm UV detection, Mobile Phase: solvent x (0.1% aqueous TFA) and solvent z (Acn). Method: Isocratic: Solvent z 20% for 25 minutes; and Column: Regis (S, S)-Whelk-01 KROMASIL^{®} 250 x 4.6mm 5µm Cat # 780101, Injection volume 20µL of an (rc1) sample at a concentration of 0.1mg/mL in 1:1 Acn/water. Retention times for 4-(4-chloro-2-oxopyridin-1(2H)-yl)-2-methyl-2-(methylsulfonyl)butanoic acid (rc2): R-enantiomer (R2, 8.98 minutes) and 9.58 minutes for the S-enantiomer.

### Preparation of Reactants, Intermediates (compounds) and Formula (1) potassium 4-chloro-2-oxo-2H-pyridin-1-ide

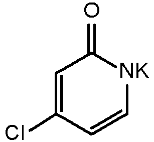

The compound was prepared by reacting 4-chloropyridin-2(1H)-one in a solvent mixture of toluene/ethanol/water in the presence of KOH at a starting temperature of about 5°C ending at about 20°C. Alternatively, the pyridin-ide can be prepared in-situ by reacting 4-chloropyridin-2(1H)-one with potassium tert-butoxide (KOtBu).

Process (rxn-1) for preparing (rc1): 3-methyl-3-(methylsulfonyl)dihydrofuran-2(3H)-one:

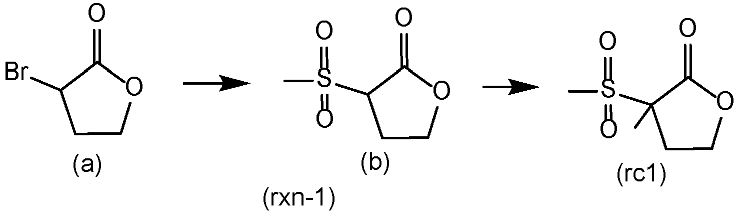

### Intermediate (b): 3-(methylsulfonyl)dihydrofuran-2(3H)-one

In a 4 neck round bottom flask (500mL) equipped with overhead stirring, heating mantle and temperature controller was charged under nitrogen with 3-bromotetrahydrofuran-2(3H)-one ((a), 100.0 g, 0.576mol, 1.00 eq). Sodium methanesulfinate (90.0 g, 85% potency, 0.750mol, 1.30 eq), Aliquat^{®}336 (1.5 g, 3.7mmol, 0.0064 eq), magnesium sulfate (6.0 g) and isopropanol (250mL). The resulted mixture was heated to 80°C. After 3 hours ¹H-NMR showed that the reaction was complete. The reaction mixture was cooled to room temperature using ice bath cooling. The resulting solid was isolated by filtration, washed with isopropanol (100mL) and dried in vacuo to give 176g of a mixture of intermediate (b) and the inorganic salts. Alternatively, the desired product (b) can be cleanly isolated by dissolution in acetone, filtering off the NaBr and solvent evaporation of the acetone filtrate. The dried solids from above were subsequently used in the next step to prepare (rc1).

### Intermediate (rc1): 3-methyl-3-(methylsulfonyl)dihydrofuran-2(3H)-one

Intermediate (b) (94 g, 0.576mol, 1.00 eq) from the solid mixture above was extracted with acetone (2 x 100mL) at 45°C (complete extraction was verified by gravimetric analysis (total weight loss 94g) and HPLC (no product detected in the cake after second extraction). Then, to the acetone extract was charged under nitrogen: potassium carbonate (325 MESH 100 g, 0.724mol, 1.26 eq), Aliquat^{®}336 (1.5 g, 3.7mmol, 0.0064 eq), dimethyl sulfate (68mL, 0.719mmol, 1.25 eq) and magnesium sulfate (6.0 g). The reaction mixture was heated to 60°C for 7 hours, ¹H NMR analysis showed 3% of (b) remaining. An additional 7g of dimethyl sulfate 10g of potassium carbonate was charged to the reactor, and heating was maintained for an additional 4 hours. The reaction mixture was filtered to remove inorganic salts. The solid was washed with acetone (100mL). The filtrate was heated to 65°C, and acetone was distilled from the reaction pot until 200mL or about 1.5 volumes of liquid remained. At this point, a solvent exchange to isopropanol (200mL) was carried out at constant reactor volume. The isopropanol solution was cooled to 35°C and seeded. Further cooling to 25°C induced crystallization. The crystals were filtered and washed with 100mL of isopropanol. Isolated 93g of (rc1) as an off white crystalline solid (91%), mp 74°C, ¹H NMR (600 MHz, CHLOROFORM-d) δ ppm 4.49 - 4.56 (m, 1 H), 4.35 - 4.44 (m, 1 H), 3.16 - 3.22 (m, 1 H), 3.08 (s, 3 H), 2.31 (dt, J=14.63, 8.83 Hz, 1 H), 1.74 (s, 3 H).

Alternatively, (rc1) can be prepared without isolation of intermediate (b) by telescoping (rxn-t) the reaction, as shown below:

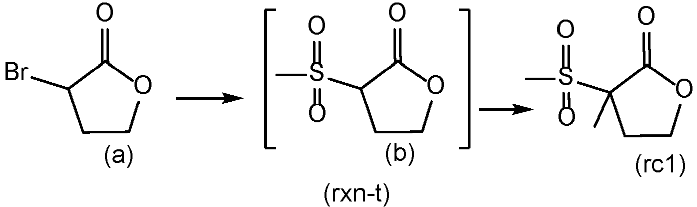

A 30 L jacketed reactor equipped with overhead stirring, temperature controller and reflux condenser was charged under nitrogen with 3-bromotetrahydrofuran-2(3H)-one (a) (2.5 kg, 96% potency, 14.4mol, 1.00 eq). Sodium methanesulfinate (2.0 kg, 85% potency, 16.7mol, 1.16 eq), Aliquat^{®}336 (30 g, 74.2mmol, 0.005 eq), magnesium sulfate (120 g, 0.07 eq) and acetone (7.5L, 3 vol). The resulted mixture was heated to reflux (56°C). After 9 hours, HPLC showed <1% of the starting material. The reaction mixture was cooled to ambient temperature and allowed to stir overnight. The reaction mixture was filtered, and the solids were washed with 200mL of acetone. A 2mL sample of the acetone filtrate was concentrated in vacuo to afford a solid (b). ¹H NMR (600 MHz, DMSO-*d₆*) δ 4.75 (dd, 1H), 4.37 (m, 2H), 2.64 (m, 2H).

To a 30L jacketed reactor equipped with overhead stirring, temperature controller and reflux condenser was added the acetone filtrate from above (8.3 kg). To this solution was added anhydrous potassium carbonate (2.73 kg, 325 mesh, 1.37 eq), dimethyl sulfate (2.27 kg, 1.25 eq) and anhydrous magnesium sulfate (120 g, 0.07 eq). The mixture was slowly heated to reflux (56°C). After 15 hours at reflux temperature, additional potassium carbonate (200 g, 0.1 eq) and dimethyl sulfate (200 g, 0.11 eq) was added, and reflux was continued for an additional 4 hours. The mixture was cooled to ambient temperature, and the reaction mixture was filtered to remove inorganic salts. The solid was washed with acetone (2L, 0.8 vol). The mother liquor was heated to 65°C, and acetone was distilled from the reaction pot until 3L of liquid remained. At this point, a solvent exchange from acetone to isopropanol (4L) was carried out at constant reactor volume. Once all the isopropanol had been dripped into the reactor, the solution was cooled to 35°C and seeded. Further cooling to 25°C induced crystallization. The mixture was further cooled to 20°C and held at that temperature for 1 hour. The crystals were filtered and washed with 2L of isopropanol. The wet cake was dried in vacuo at 40°C overnight to afford 2.0kg of (3-methyl-3-(methylsulfonyl)dihydrofuran-2(3H)-one as an off-white, crystalline solid (78%), mp 74°C, ¹H NMR (600 MHz, Chloroform-d) δ ppm 4.49 - 4.56 (m, 1 H), 4.35 - 4.44 (m, 1 H), 3.16 - 3.22 (m, 1 H), 3.08 (s, 3 H), 2.31 (dt, J=14.63, 8.83 Hz, 1 H), 1.74 (s, 3 H).

A separate synthesis for preparing (rc1) from intermediate (a), using the procedure described above but without the use of the dehydrating agent, magnesium sulfate, during the sulfonylation and methylation reactions provided a yield of 64% for (rc1).

A separate synthesis for preparing intermediate (b) from intermediate (a) without magnesium sulfate and replacing the acetone with isopropanol provided a yield of 83% of intermediate (b). In a 2-neck round bottom flask equipped with magnetic stir bar, heating mantle and temperature controller was charged under nitrogen with 3-bromotetrahydrofuran-2-one ((a), 4.0 g, 24.2mmol, 1.00 eq), sodium methanesulfinate (3.0 g, 28.3mmol), 1.2 eq), Aliquat^{®}336 (0.12 g, 0.50mmol, 0.02 eq), isopropanol (12mL). The resulted mixture was heated to 80°C. After 3 hours ¹H-NMR (600 MHz, DMSO-d6) indicated that the reaction was complete. The reaction mixture was cooled to ambient temperature using an ice bath. The resulting solid was isolated by filtration, washed with isopropanol (5mL) and concentrated in vacuo to afford a white solid. The solid was reslurried in acetone (20mL) and filtered to remove sodium bromide. The mother liquor was concentrated in vacuo to afford a white solid, 3.3g (83%), Table 1.

Similar preparations of the sulfonyl lactone (b) from intermediate (a) without the use of magnesium sulfate were performed using different solvents which are shown in Table 1.

**Table 1. Varying Solvents to Prepare Intermediate (b).**

| Solvent | Yield (%) |
|---|---|
| isopropanol | 83 |
| ethanol (3 vol) | 72 |
| methanol (3 vol) | 67 |
| acetonitrile (3 vol) | 68 |

Small scale preparations of the methylsulfonyl lactone (rc1) from intermediate (b) as described above were conducted without the dehydrating agent magnesium sulfate while also changing the base, methylating agent and/or solvent. The (rc1) yields from each of these reactions is shown in Table 2.

**Table 2. Varying Reactants and/or Solvents to Prepare (rc1).**

| Base | Solvent | Methylating Agent | Yield (%) |
|---|---|---|---|
| K₂CO₃ | acetone | dimethyl sulfate | 79 |
| K₂CO₃ | acetone | methyl tosylate (1.25 eq) | 59 |
| K₂CO₃ | acetone | iodomethane | 97 |
| Na₂CO₃ | acetone | iodomethane | 50 |
| K₃PO₄ (tribasic, 1.25 eq) | acetone | iodomethane | 70 |
| Na₂CO₃ | 2-butanone | iodomethane | 75 |
| Na₂CO₃ | acetonitrile | iodomethane | 95 |

### General Procedure for lactone opening: Synthesis of intermediate 4-(4-chloro-2-oxopyridin-1(2H)-yl)-2-methyl-2-(methylsulfonyl)butanoic acid (rc2)

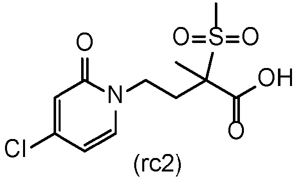

The reactions were performed in a 125mL Mettler Easy Max^{®} reactor (stir rate 400 RPM) or a 40mL scintillation vial with magnetic stirrer under nitrogen. Potassium tert-butoxide (9.89 g, 87.3mmol, 1.01 eq) was dissolved in anhydrous dimethyl sulfoxide (23mL) and added to 4-chloro-2-hydroxypyridine (i.e, 4-chloropyridin-2(1H)-one (tautomer)) (11.2 g, 86.5mmol, 1.00 eq) in anhydrous dimethyl sulfoxide (11mL) while maintaining internal temperture between 18-27°C. After 15 minutes, a mixture of (rc1), 16.5 g, 92.6mmol, 1.07 eq) and benzyltriethylammonium bromide (1.21 g, 4.44mmol, 0.0514 eq) was added in one portion. The reaction mixture was heated to 85°C for 2 hours and sampled for HPLC analysis. HPLC chromatogram showed 30.7% chloropyridone, 65.2% (rc2) and 4.1% O-alkylated product, 5-((4-chloropyridin-2-yl)oxy)-2-methyl-2-(methylsulfonyl)pentanoic acid. Then the reaction mixture was aged at 85°C for 17 hours (total reaction time 19 hours) and re-sampled. HPLC indicated 5% chloropyridone, 88.1% (rc2), and 2.4% of an O-alkylated compound. While hot, the reaction mixture was treated with isopropyl acetate (50mL) and transferred into 400mL reactor. The mixture was treated with additional isopropyl acetate (100mL) stirred for 1 hour at jacket temperature of 60°C. The jacket was cooled to 20°C over 1 hour and aged for 1 hour. The resulting solids were isolated by filtration, washed with isopropyl acetate (2 x 30mL) and dried overnight at 50°C to give 25.75g (86%) of (rc2) (potassium salt) as an off-white solid.

### Synthesis of free carboxylic acid (rc2)

To 5.0 grams of the above potassium salt was dissolved in water (15mL) and treated with methyl alcohol (10mL) and Me-THF (4mL). Then, 1.8mL of concentrated HCl was charged. The pH of the slurry was about 1. The slurry was stirred at room temperature for 2 hours and then cooled to 0-5°C and stirred at that temperature for one hour. The slurry was filtered and washed with water:methanol (8:2) and then dried in vacuo with a nitrogen bleed to give 4.04 grams of (rc2) (90.8%). ¹H NMR (600 MHz, DMSO-d₆) δ ppm 1.55 (s, 3 H) 2.14 (ddd, J=13.39, 10.18, 4.86 Hz, 1 H) 2.42 (ddd, J=13.34, 10.22, 5.96 Hz, 1 H) 3.14 (s, 3 H) 3.92 (ddd, J=12.84, 10.18, 5.96 Hz, 1 H) 4.03 (ddd, J=12.88, 10.22, 4.95 Hz, 1 H) 6.37 (dd, J=7.34, 2.38 Hz, 1 H) 6.52 (d, J=2.38 Hz, 1 H) 7.76 (d, J=7.15 Hz, 1 H) 13.85 (br s, 1 H). ¹³C NMR (151 MHz, DMSO-*d*₆) δ ppm 14.70, 31.65, 36.91, 44.20, 69.36, 106.82, 117.74, 139.97, 145.66, 160.33, and 169.18.

Alternatively, (rc2) was prepared according to the following procedure. To a 400mL reactor was charged under nitrogen: PEG 400 (15.6 g, 38.6mmol, 0.20 equiv) 2-methyl-2-butanol (175mL, 7V) and 4-chloro-2-hydroxypyridine (25.0 g, 193mmol, 1.0 equiv). The jacket temperature was adjusted to 20°C and potassium tert-butoxide (23.0 g, 201mmol, 1.041 eq) was added while stirring using mechanical stir (300-600 rpm). Note: The internal temperature rose from 19°C to 42°C. To control the exotherm at scale, addition of portion wise KOtBu was added. The reaction mixture was aged for 30-45 minutes at 40-45°C; then 3-methyl-3-methylsulfonyl-tetrahydrofuran-2-one (38.6 g, 212mmol, 1.10 equiv) was charged (one portion). The reactor jacket was heated to 100°C over 20 minutes and maintained for 16-20 hours (internal temperature was 95-98°C). The reaction was deemed complete when 4-chloro-2-hydroxypyridine was below 5% a/a (HPLC or NMR). Then reaction mixture was cooled to 50-60°C, diluted with water (63mL, 2.5 V) and the pH adjusted to 1-2 using concentrated HCl (about 20mL). Then, the reaction mixture was cooled to 0°C over 2 hours, aged for 1 hour and the resulting solid isolated by filtration. The product was washed with deionized water (2 x 40mL) and dried to constant weight at 70°C in high vacuum to give 46.5g (78.3%) of 4-(4-chloro-2-oxopyridin-1(2H)-yl)-2-methyl-2-(methylsulfonyl)butanoic acid as off white crystalline solid. HPLC purity 98.4% a/a at 230 nm. 1H NMR (600 MHz, DMSO-d6) δ ppm 1.55 (s, 3 H) 2.14 (ddd, J=13.39, 10.18, 4.86 Hz, 1 H) 2.42 (ddd, J=13.34, 10.22, 5.96 Hz, 1 H) 3.14 (s, 3 H) 3.92 (ddd, J=12.84, 10.18, 5.96 Hz, 1 H) 4.03 (ddd, J=12.88, 10.22, 4.95 Hz, 1 H) 6.37 (dd, J=7.34, 2.38 Hz, 1 H) 6.52 (d, J=2.38 Hz, 1 H) 7.76 (d, J=7.15 Hz, 1 H) 13.85 (br s, 1 H). 13C NMR (151 MHz, DMSO-d6) δ ppm 14.70, 31.65, 36.91, 44.20, 69.36, 106.82, 117.74, 139.97, 145.66, 160.33, and 169.18.

### Classical Resolution of (rc2) to (R2)

To a slurry of (rc2) (1.0 eq) in methanol (5mL/g) was slowly added (30-60 minutes) a solution of (S)-(÷)-α-methylbenzylamine (1.05 eq) in methanol (1 vol) while maintaining the temperature between 55°C and 65°C. Reaction was heated at 65°C for 30 minutes, followed by slow cooling to 60°C. The mixture was seeded and cooled to 0°C over a period of 5 hours and aged for 2 hours. Slurry was filtered and washed with IPA (2 x 1 vol) and dried under vacuum at 55°C for overnight to obtain the desired diastereomeric salt (18.95 kg; 44% yield and 98.93% purity).

### Synthesis of (R2) from (R1)

A sample of (R2) was prepared using the general procedure shown above for (rc2) to (R2) starting from 4-chloro-2-hydroxypyridine (1.14 g, 8.80mmol) and (R1), 3-methyl-3-methylsulfonyl-tetrahydrofuran-2-one (1.65 g, 9.26mmol). Isolated 1.63g of (R2) (60%). HPLC purity 96.4% (achiral); Chiral HPLC *R_{f}* 8.98 minutes, purity 99.9%.

### Chiral Chromatography to Separate (R1) from (rc1), (R)-3-methyl-3-(methylsulfonyl)dihydrofuran-2(3H)-one and (S)-3-methyl-3-(methylsulfonyl)dihydrofuran-2(3H)-one

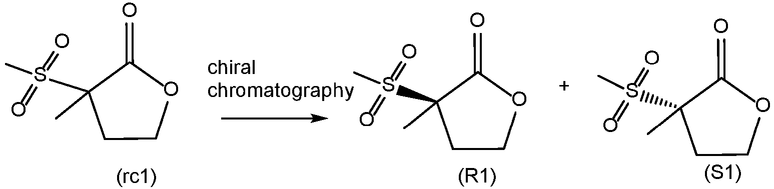

A total of 5g of racemate, (rc1), was passed through a preparative Sepiatec SFC 250 instrument equipped with a 30x250mm 5µ Daicel Chiralpak^{®} AD-H column with UV detection at 230nm. The column was eluted with mobile phase A (CO₂) and mobile phase B (MeOH); isocratic 25%B; 90/mL/minute, 140bar back pressure. The (R) enantiomer was the first eluting peak at 2.4 minutes and the second eluting peak, (S), eluted at 2.8 minutes. Eluant was evaporated using a rotary evaporator and the residue was lyophilized from a mixture of acetonitrile/water. The first peak (2.15 g, 43%) had an HPLC chiral purity of >99% while the second peak (2.32 g, 46%) had an HPLC chiral purity of 96.0%. The peak assignment was performed by comparing the retention time of each peak with the retention time of its respective reference standard. Reference standards:
- (R)-3-methyl-3-(methylsulfonyl)dihydrofuran-2(3H)-one optical rotation - 0.198 [α]²⁰₅₈₉ = -9.960° (2, 0.5, MeOH) and melting point of 94°C
- (S)-3-methyl-3-(methylsulfonyl)dihydrofuran-2(3H)-one optical rotation +0.184 [α]²⁰₅₈₉ = + 9.250° (2, 0.5, MeOH) and melting point of 92°C

### Synthesis of (S)-1-phenylethan-1-aminium (R)-4-(4-chloro-2-oxopyridin-1(2H)-yl)-2-methyl-2-(methylsulfonyl)butanoate

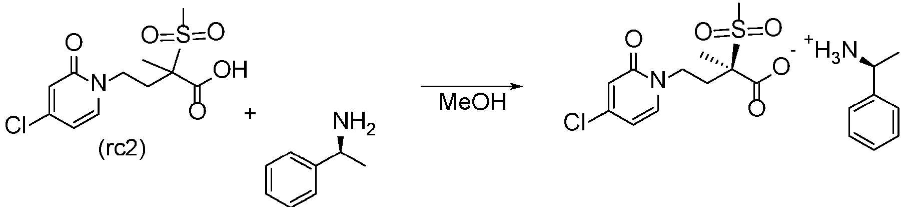

To a 400mL reactor was charged 4-(4-chloro-2-oxopyridin-1(2H)-yl)-2-methyl-2-(methylsulfonyl)butanoic acid (rc2), 49.95 g, 162.3mmol, 1.0 eq) followed by methanol (200mL, 4 vol). The reaction mixture (slurry) was heated to 55-60°C (target 60°C internal temperature) over a period of 0.5 to 1.5 hours. In a separate reactor was prepared a solution of (S)-α-methylbenzylamine (20.76, 171.3mmol, 1.056 eq) in methanol (25mL, 0.5 vol). The solution of (S)-α-methylbenzylamine in methanol was transferred to the reactor containing the racemate over a period of 30 minutes. After 80% of the solution was added the slurry becomes a clear solution. The reactor and the transfer line was rinsed with methanol (12mL, 0.25 vol) and added to the reaction mixture. Then the reaction mixture was heated to reflux (65°C) over a period of 0.5 to 1 hour, aged at 65°C for 0.5 to 1 hour, and cooled to 60°C over 30 minutes. The reaction mixture was seeded with (S)-1-phenylethan-1-aminium (R)-4-(4-chloro-2-oxopyridin-1(2H)-yl)-2-methyl-2-(methylsulfonyl)butanoate (0.250g 0.5% of racemic input). Note: If seeds dissolved, cool to 55°C over 30 minutes, and reseed the mixture with (S)-1-phenylethan-1-aminium (R)-4-(4-chloro-2-oxopyridin-1(2H)-yl)-2-methyl-2-(methylsulfonyl)butanoate (0.250g 0.5% of racemic input). The reaction mixture was aged for 1-3 hours at 60°C to initiate crystallization and cooled to -5 to 5°C (target 0°C) over 4-10 hours (target 5 hours). The reaction mixture was aged at -5°C for 1 hour and the resulting solids isolated by filtration. The product was washed with IPA (2x 25mL, 0.5 vol each) and dried under vacuum at 55-65°C for not less than 10 hours to give 30.6g of (S)-1-phenylethan-1-aminium (R)-4-(4-chloro-2-oxopyridin-1(2H)-yl)-2-methyl-2-(methylsulfonyl)butanoate as white crystalline solid (44%).

### Synthesis of (R)-4-(4-chloro-2-oxopyridin-1(2H)-yl)-2-methyl-2-(methylsulfonyl)butanoic acid, (R2),

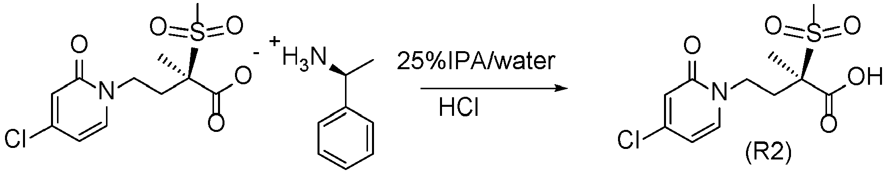

To a 30L glass reactor was charged with (S)-1-phenylethan-1-aminium (R)-4-(4-chloro-2-oxopyridin-1(2H)-yl)-2-methyl-2-(methylsulfonyl)butanoate (2.655 kg, 5.757mol, 1.0 eq) and 25% IPA in water (15L, 5.6 vol). The resulting mixture was heated to 35 to 45°C (target 40°C) over 30 minutes and while maintaining temperature concentrated HCl (0.264L/kg, 1.25 eq, ~ 11N) was charged slowly until the pH was between 1.0 and 2.0. Note in the current example 662mL of concentrated HCl was added to give a pH of 1.4. The mixture was aged for 0.5 hours at about 45°C, then cooled to 0°C over a period of 2 hours and aged at 0°C for 0.5 hours. The resulting solid was isolated by filtration, washed with water (2x 2L, 0.75 vol each) and dried under vacuum at 65°C with nitrogen bleed to give 1.785kg of (R)-4-(4-chloro-2-oxopyridin-1(2H)-yl)-2-methyl-2-(methylsulfonyl)butanoic acid, (R2) (100%). Optical rotation = +0.425 [α]²³₅₈₉ = +42.500° (2, 0.5, MeOH)

### Borylation: Synthesis of 2-(4-(1,3,2-dioxaborolan-2-yl)phenyl)-2H-1,2,3-triazole

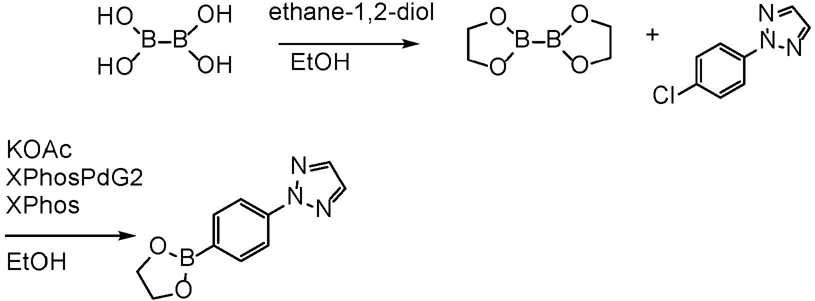

In a suitable vessel (12L flask, vessel 1) equipped with mechanical stirrer and temperature indicator was prepared a solution of 2,2'-bi(1,3,2-dioxaborolane) in ethanol by mixing tetrahydroxydiboron (0.398 kg, 4.306mol, 1.50 eq), ethylene glycol (0.481 kg, 7.753mol, 2.699 eq) and 200 proof ethyl alcohol (4L, 8 vol). The slurry was stirred without heating at about 20-25°C until clear solution was observed (NLT 3 hours). The solution may be stored under nitrogen purge at ambient temperature up to 24 hours. In a separate vessel (vessel 2) was charged ethanol (4.8L, 9.5 vol), acetic acid (84.5g 1.4mol, 0.489 eq) and the resulting solution degassed for about 10 minutes using nitrogen. Then, 2-(4-chlorophenyl)-2H-1,2,3-triazole (0.516 kg, 2.872mol,1.000 eq), predried potassium acetate (0.691 kg, 6.97mol, 2.43 eq), XPhosG2 (4.43 g. 5.52mmol 0.002 eq) and XPhos (5.37 g, 11.0mmol, 0.004 eq) were added. The reaction mixture was heated to about 50-60°C, aged for 15- 30 minutes and then cooled to attain an internal temperature of about 40-50°C. The solution in vessel 1 was degassed with nitrogen for about 10 minutes and then transferred using nitrogen pressure to the reaction mixture in vessel 2 while maintaining an internal temperature of about 45-60°C (Note: reaction is exothermic). The transfer line was rinsed with degassed ethanol (1 vol). Then the reaction mixture was aged at an internal temperature of 50-60°C for about 1 hour and sampled to check reaction conversion. The reaction was deemed complete when the residual aryl chloride was below 0.5% (a/a). If residual aryl chloride is above 0.5% a.a, additional tetrahydroxydiboron (1.5 eq) with respect to % of unreacted 2-(4-chlorophenyl)-2H-1,2,3-triazole may be added. Note: The limiting reagent for this step is 2-(4-chlorophenyl)-2H-1,2,3-triazole. Alternatively, the boylation step can be conducted in a one-pot process.

### Alternatively, 2-[4-(4,4,5,5-tetramethyl-1,3,2-dioxaborolan-2-yl)phenyl]-2H-1,2,3-triazole can be prepared for the Suzuki coupling reaction

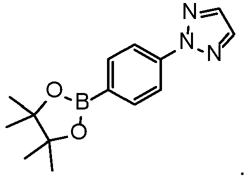

The complex, [1,1'-Bis(diphenylphosphino)ferrocene]dichloropalladium(II), and dichloromethane (280mg, 0.34mmol) was added to a mixture of 2-(4-bromophenyl)-2H-1,2,3-triazole (255mg, 1.14mmol), 4,4,4',4',5,5,5',5'-octamethyl-2,2'-bi-1,3,2-dioxaborolane (350mg, 1.38mmol), and potassium acetate (340mg, 3.46mmol) in 1,4-dioxane (10mL). The reaction mixture was heated to 80°C and stirred at this temperature overnight. The reaction mixture was allowed to cool to room temperature and diluted with ethyl acetate (30mL) and brine (30mL). The mixture was filtered through celite (~1 inch). The organic layer was separated and the aqueous layer was extracted with ethyl acetate (2x30mL). The organic layers were combined, dried (MgSO₄), filtered, and concentrated in vacuo. The crude material was purified via flash chromatography using an Analogix SF15-12g column and a gradient of 0-10% ethyl acetate in n-heptane to afford an orange solid (240.6mg, 78%). 1 H NMR (400 MHz, CDCl3) δ 8.10 (d, J = 8.59 Hz, 2H), 7.94 (d, J = 8.59 Hz, 2H), 7.83 (s, 2H), 1.36 (s, 12H). LCMS: m/z 272.4 [M+H]+ .

### Suzuki Coupling: Synthesis of (R)-4-(4-(4-(2H-1,2,3-triazol-2-yl)phenyl)-2-oxopyridin-1(2H)-yl)-2-methyl-2-(methylsulfonyl)butanoic acid (2.1)

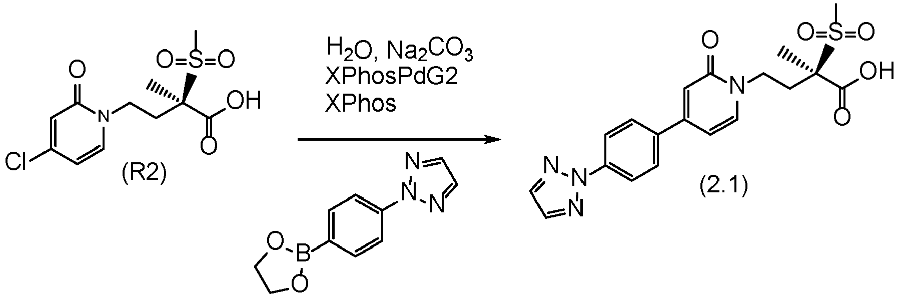

In a separate reactor (vessel 3), was charged deionized water (6L, 6.9 vol), sodium carbonate (0.745g, 7.036mol 2.5 eq), (R)-4-(4-chloro-2-oxopyridin-1(2H)-yl)-2-methyl-2-(methylsulfonyl)butanoic acid (0.866 kg, 2.814mol, 1.0 eq) and stirred at about 20-30°C until a solution was observed. The sodium carboxylate solution was degassed for about 10 minutes using nitrogen. Then, the degassed solution was added to the reaction mass in vessel 2 containing the 2-(4-(1,3,2-dioxaborolan-2-yl)phenyl)-2H-1,2,3-triazole while maintaining internal temperature of about 50-60°C. After addition was completed the original vessel and the transfer lines were rinsed with degassed deionized water (1 vol). The reaction mixture was heated to about 70-80°C, aged for 3 hours and sampled for HPLC analysis. The reaction was considered complete if residual 2-(4-(1,3,2-dioxaborolan-2-yl)phenyl)-2H-1,2,3-triazole was below 0.5% a/a. If the residual 2-(4-(1,3,2-dioxaborolan-2-yl)phenyl)-2H-1,2,3-triazole is above 0.5% a.a; additional (R)-4-(4-chloro-2-oxopyridin-1(2H)-yl)-2-methyl-2-(methylsulfonyl)-butanoic acid (0.025 eq) may be added. The reaction mixture was cooled to about 50-60°C and 2-methyl tetrahydrofuran (3.2L, 3.7 vol) was charged while internal temperature maintained between 50-60°C. The mixture was further cooled to attain and internal temperature of about 40-50°C. Then, concentrated hydrochloric acid (961mL, 1.11 vol) was slowly charged while maintaining an internal temperature of about 40-50°C. If solids are not observed, then charge additional concentrated hydrochloric acid (0.1 vol). The reaction mixture was aged for about 1 hour at internal temperature of about 40-50°C and then the pH was adjusted to 1 using concentrated HCl (961mL, 1.1 vol) while maintaining an internal temperature of about 40-50°C. Note: the pH must be NMT 1. Finally, the batch was cooled to about 0-10°C and aged for not less than 1 hour. The product was isolated by filtration, the wet cake was washed with a mixture (1:1 v/v) of ethanol/deionized water (4L) followed by deionized water (1.65L). The product was transferred to a dryer and dried under vacuum at jacket temperature at no more than 65°C until LOD was below 0.5 w/w% to afford 1.153g of (R)-4-(4-(4-(2H-1,2,3-triazol-2-yl)phenyl)-2-oxopyridin-1(2H)-yl)-2-methyl-2-(methylsulfonyl)butanoic acid (2.1) as a light beige solid (98%). ¹H-NMR (600 MHz, DMSO-d6) δ ppm 1.55 (s, 3 H) 2.14 (m, 1 H) 2.42 (m, 1 H) 3.14 (s, 3 H) 3.92 (m, 1 H) 4.03 (m, 1 H) 6.37 (dd, 1 H) 6.52 (d, 1 H) 7.76 (d, 1 H) 13.85 (br s, 1 H).

### Acid chloride formation: synthesis of (R)-4-(4-(4-(2H-1,2,3-triazol-2-yl)phenyl)-2-oxopyridin-1(2H)-yl)-2-methyl-2-(methylsulfonyl)butanoyl chloride (2.2)

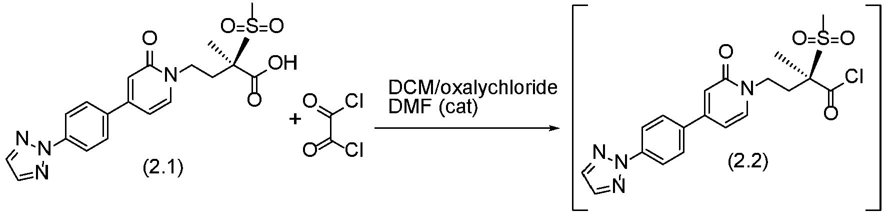

To a dry 30L jacketed glass reactor connected to a basic scrubber (the reaction will off gas HCl, CO₂ and CO) was charged at 20°C under nitrogen with dichloromethane (10L, 10 vol) followed by oxalyl chloride (0.908 kg, 7.150mol, 2.980 eq) and N,N-dimethylformamide (10mL, 129mmol, 0.0538 eq). Then, slowly charged with (R)-4-(4-(4-(*2H*-1,2,3-triazol-2-yl)phenyl)-2-oxopyridin-1(*2H*)-yl)-2-methyl-2-(methylsulfonyl)butanoic acid, 2.1 (1.000 kg, 2.401mol, 1.0 eq) (NLT 30 minutes) while maintaining internal jacketed temperature of about 20-35°C. Note: gas evolution is expected. The reaction mixture was aged for 90-120 minutes at 20-40°C (target 30°C) and sampled for HPLC analysis. The reaction was deemed complete when carboxylic acid was < 5% (target < 3%). Note: The conversion is determined by quenching 2 drops raw material with 2mL of anhydrous methanol. The excess oxalyl chloride/DCM was distilled off under atmospheric pressure to a thick slurry (minimal volume 1-2L) and then heptane (5L, 5 vol) was charged). The distillation was continued until jacketed internal temparturre reaches 96-98°C (thick slurry). Note: The mixture of acid chloride in heptane may be stored overnight under nitrogen at room temperature. Final volume in the tank was about 1-2L. Alternatively, excess oxalyl chloride can be removed via series of decantation/DCM dilutions or by adding a controlled amount of water at low temperature (-5 to 5°C).

### Amide bond formation - Synthesis of (2R)-4-(4-(4-(2H-1,2,3-triazol-2-yl)phenyl)-2-oxopyridin-1(2H)-yl)-2-methyl-2-(methylsulfonyl)-N-((tetrahydro-2H-pyran-2-yl)oxy)butanamide, (2.3)

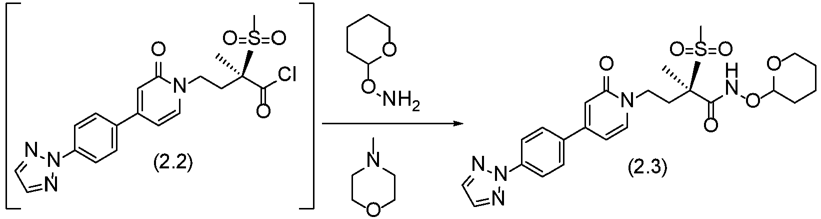

The above reaction was cooled to 20-30°C, diluted with dichloromethane (7.3L, 7.3 vol) and then cooled to -5 to 5°C. To the cold reaction mixture was slowly charged 4-methylmorpholine (0.777 kg, 7.682mol, 3.199 eq) while keeping the temperature below 10°C. Note: Especially important, to control moisture; 4-methylmorphine should be reagent grade or better. In a separate reactor a solution of O-(tetrahydro-2H-pyran-2-yl)hydroxylamine (THP-ONH₂; 0.337 g, 2.885mol, 1.202 eq) in dichloromethane (3.4L, 3.4 vol) was prepared. The moisture content of the solution must be below 500ppm if higher than 500 ppm azeotropic drying must be performed as follows: distill 40% of the batch volume and then add anhydrous DCM, sample for Karl Fisher titration, repeat until moisture is below 500 ppm. The THP-ONH₂ solution was transferred slowly over 40 minutes to acid chloride while keeping the temperature below 10°C (target -5 to 5°C). Then, transfer lines were rinsed with dichloromethane (1L, 1 vol). The reaction mixture apparent pH was checked using pH paper. If not between 7-10, additional 4-methyl-morpholine must be charged until apparent pH is adjusted to the desired range. Then, the reaction mixture was aged to -5 to 5°C for 1-2 hours; warmed to 20-30°C over 1-3 hours and kept at 20-30°C for not less than 30 minutes. The reaction mixture was sampled to check conversion using HPLC. The residual acid chloride was measured by converting the acid chloride into methyl ester. The reaction was deemed complete when the acid chloride was below 0.5%. Then, small amount of water (330mL) was charged, and the reaction mixture aged for not less than 30 minutes. The product was isolated as follows: The reaction mixture was concentrated by distillation (reactor jacket temperature 60°C) at atmospheric pressure to remove 7L of DCM and then diluted with methanol (8L, 8 vol). Distillation was continued at atmospheric pressure and the jacket temperature was 60-80°C to remove 7-8 volumes of DCM/MeOH. Finally, methanol (5L, 5 vol) was charged and distillation at atmospheric pressure continued at jacket temperature of 80-90°C, until the internal temperature reached 65-70°C (target 67°C). About 5L of distillate was collected. The reaction mixture was then cooled to -5 to 5°C (target 0°C) in not less than 2 hours, aged for 1 hour at target temperature and the resulting solid isolated by filtration. The product was washed with methanol (3 x 1L), pooled, dried for 30 minutes and dried under vacuum at 50-60°C to give 1.223kg of product (98.8% yield) HPLC purity 95.9%. ¹H-NMR (600 MHz, DMSO-d6) δ ppm 1.53 (m, 3 H) 1.59 (d, 3 H) 1.69 (m, 3 H) 2.14 - 2.27 (m, 1 H) 2.37-2.48 (m, 1 H) 3.11 (d, 3 H) 3.44 - 3.63 (m, 1 H) 3.65 - 3.93 (m, 1 H) 3.96 - 4.11 (m, 1 H) 4.11 - 4.32 (m, 1 H) 4.98 (br s, 1 H) 6.61 -6.77 (m, 1 H) 6.79 (m, 1 H) 7.79 (dd, 1 H) 7.96 (m, 2 H) 8.04 - 8.16 (m, 2 H) 8.18 (s, 2 H) 11.63 (br s, 1 H)

### Synthesis of Formula (1): (R)-4-(4-(4-(2H-1,2,3-triazol-2-yl)phenyl)-2-oxopyridin-1(2H)-yl)-N-hydroxy-2-methyl-2-(methylsulfonyl)butanamide

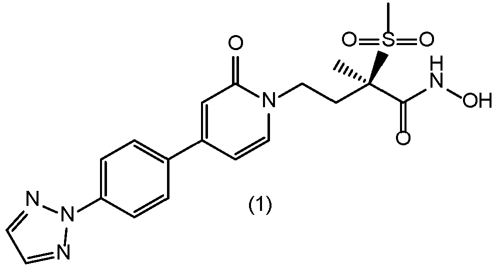

In a 30L glass reactor was charged with (2R)-4-(4-(4-(2H-1,2,3-triazol-2-yl)phenyl)-2-oxopyridin-1 (2H)-yl)-2-methyl-2-(methylsulfonyl)-N-((tetrahydro-2H-pyran-2-yl)oxy)butanamide, 2.3, (2104 g, 4.08mol, 1.00 eq), 2-propanol (21L, 10 vol) and HCl (1mol/L) in water (6.3L, 6.30mol, 1.54 eq). Alternatively, 0.025-1 equivalents of sulfuric acid in the reaction solvent or about 1 equivalent of maleic acid in the reaction solvent can also be used. The resulting mixture was heated to 60-70°C while stirring at a rate of about 210 rpm and aged to 62-63°C for about 40-60 minutes. Then, the reaction was sampled to confirm reaction completion (i.e., <0.5%). If not, continue reaction up to about 4 hours. After reaction completion, the reaction mixture was cooled to 20° (±5°C) over 2-4 hours and aged at 20°C for 1-2 hours. The final product (Formula (1)) was isolated by filtration, washed with 2-propnaol (4L, 2 vol), water (2 x 1.5L) and dried at 55-65°C using house vacuum to give 1.665kg (95%). ¹H-NMR (600 MHz, DMSO-*d*₆) δ ppm 1.59 (s, 3 H) 2.18-2.21 (m, 1 H) 2.40 - 2.48 (m, 1 H) 3.11 (s, 3 H) 3.76-3.79 (m, 1 H) 4.12-4.16 (m, 1 H) 6.71 (dd, 1 H) 6.78 (d, 1 H) 7.79 (d, 1 H) 7.85 - 8.04 (m, 2 H) 8.04 - 8.15 (m, 2 H) 8.17 (s, 2 H) 9.24 (s, 1 H) 11.15 (s, 1 H).

Alternatively, final THP removal can be accomplished in solvents like ethanol, isopropanol, acetone, acetic acid/tetrahydrofuran in the presence of a strong acid (e.g., HCl or HBr); or the Formula (1) compound can be prepared in accordance with the procedural steps described in PCT publication WO2019/178305 with the iodo pyridinone or replacing with chloro pyridinone; or in accordance with the procedural steps described in US Patent No. 8,664,401.

### Crystal Forms

A number of polymorphic forms of (R)-4-(4-(4-(2H-1,2,3-triazol-2-yl)phenyl)-2-oxopyridin-1 (2H)-yl)-N-hydroxy-2-methyl-2-(methylsulfonyl)-butanamide were prepared (Forms 1, 2 and 3) as API slurries in a number of protic and aprotic solvents. With the exception of acetonitrile, methyl ethyl ketone, and tetrahydrofuran, the use of aprotic solvents like ethyl, butyl or propyl acetate result in Form 1. With the exception of water, the use of protic solvents such as butyl, propyl and ethyl alcohol favor the formation of Form 2. An unstable Form 3 was also noted. The presence of water in alcohols increased the rate of Form 2 isolation from solution. Using pure water as a solvent produced primarily Form 1 in early experiments but in scale up experiments it was found that the solid isolated from pure water and rapidly analyzed by XRD was primarily Form 2. The three forms were structurally related and were solvated and/or hydrated. The tendency of this API to form solvates indicates that other crystalline solvates, not described here, may form when the API is dissolved in solution and then crystallized by means such as anti-solvent addition, cooling, or solvent evaporation.

The relative stability of the three isolated solid forms appears to be different as suspensions compared to isolated solids. As isolated solids Form 1 is likely the most stable form. As suspensions, the relative stability of these solvates is dependent on the solvent or solvent/water mixture that is present. Form 2 may be more stable than Form 1 in solution under specific conditions but Form 2 is meta-stable relative to Form 1 as an isolated solid. Form 3 appears to be a kinetic form, is meta-stable and converts to Form 2. No XRD patterns of Form 3 plus Form 1 were observed, suggesting that Form 3 converts to Form 2 and Form 2 converts to Form 1.

The powder X-ray diffraction (XRD) patterns (Figure 1 (Forms 1,2,3)) and relative peak positions, intensities and d-spacings of Form 1 and Form 2 (Tables 3a and 3b) are very similar which strongly suggests that these three phase Forms share a substantially similar set of structural motifs. It is very likely that these three solvated solids are nearly isostructural and differ in subtle ways that are linked to the way solvent or water are packed into the solvation sites in the structure. There is an increasing complexity of the XRD patterns with Form 1 being the most complex (most peaks) and Form 3 having the fewest peaks. This pattern complexity (F1>F2>F3) parallels the apparent stability of the dried isolated samples. Out of 384 solvent mixtures, a total of 90 samples were mixtures rather than pure phase Forms.

As will be appreciated by the skilled crystallographer, the relative intensities, positions and spacings of the various peaks reported in the Tables (3a and b) and Figure (1) herein may vary due to a number of factors such as orientation effects of crystals in the X-ray beam or the purity of the material being analyzed or the degree of crystallinity of the sample. The XRD peak positions may also shift for variations in sample height but the peak positions will remain substantially as defined in the Tables. The skilled crystallographer also will appreciate that measurements using a different wavelength will result in different shifts according to the Bragg equation - nA, = 2ci sinΘ. Such further XRD patterns generated by use of alternative wavelengths are considered to be alternative representations of the XRD patterns of the crystalline materials.

The X-ray diffractograms were obtained on a Scintag X1 powder diffractometer equipped with a Peltier cooled solid state detector. Data were collected between 4 and 40 °2θ using a 0.03° step size and data acquisition time was 45 to 60 minutes for sample characterization. Data collection for solids isolated from the bulk of the small scale preliminary experiments was at a 0.04° step size over 15 minutes between 7 and 37 degrees 2-theta. Copper Kα radiation (λ = 1.54056 Å) was utilized and the tube voltage and amperage were set to 45 kV and 40 mA, respectively. Instrument calibration was performed using a quartz reference standard.

Thermal data cannot clearly differentiate forms that can be readily identified based on the XRD pattern. The thermal data indicates that all three solid forms are solvated, but the solvent content is variable. All samples, regardless of Form, showed the same DSC melting point endotherm maximum peak (≈195°) within 1°C. Thermal analyses (Differential Scanning Calorimetry (DSC)) of these samples were performed on a TA Instruments DSC 2920. The sample was placed into an aluminum DSC pan, and the weight accurately recorded. The lid and pan were fitted together but not crimped so that the volatile component could escape. The sample was heated at 10°C per minute to 300°C.

**Table 3a. Powder X-Ray Diffraction Peak Data for Form 1**

| 2-theta° | d-spacing (A°) | Intensity | 2-theta° | d-spacing (A°) | Intensity |
|---|---|---|---|---|---|
| 5.108 | 17.286 | 2008 | 24.754 | 3.594 | 458 |
| 5.835 | 15.134 | 4171 | 25.094 | 3.546 | 788 |
| 7.199 | 12.270 | 292 | 25.391 | 3.505 | 1246 |
| 8.902 | 9.926 | 1246 | 25.830 | 3.446 | 602 |
| 9.730 | 9.083 | 2159 | 26.129 | 3.408 | 356 |
| 10.248 | 8.625 | 201 | 26.455 | 3.366 | 642 |
| 12.445 | 7.107 | 2159 | 26.961 | 3.304 | 496 |
| 12.977 | 6.817 | 1947 | 27.289 | 3.265 | 484 |
| 13.925 | 6.354 | 414 | 27.527 | 3.238 | 333 |
| 14.313 | 6.183 | 612 | 28.103 | 3.173 | 786 |
| 15.272 | 5.797 | 3433 | 28.536 | 3.125 | 425 |
| 15.384 | 5.755 | 3301 | 28.994 | 3.077 | 231 |
| 15.749 | 5.622 | 409 | 29.389 | 3.037 | 348 |
| 16.101 | 5.500 | 738 | 29.641 | 3.011 | 420 |
| 16.905 | 5.240 | 16069 | 29.877 | 2.988 | 435 |
| 17.588 | 5.039 | 496 | 30.380 | 2.940 | 262 |
| 17.891 | 4.954 | 736 | 30.800 | 2.901 | 307 |
| 18.111 | 4.894 | 853 | 31.109 | 2.873 | 193 |
| 18.673 | 4.748 | 672 | 31.680 | 2.822 | 215 |
| 19.048 | 4.656 | 379 | 32.266 | 2.772 | 205 |
| 19.433 | 4.564 | 488 | 32.915 | 2.719 | 194 |
| 19.680 | 4.507 | 1483 | 33.332 | 2.686 | 498 |
| 20.604 | 4.307 | 289 | 34.261 | 2.615 | 156 |
| 21.184 | 4.191 | 1699 | 34.800 | 2.576 | 179 |
| 21.764 | 4.080 | 1235 | 35.255 | 2.544 | 174 |
| 22.268 | 3.989 | 569 | 35.612 | 2.519 | 145 |
| 22.786 | 3.900 | 465 | 36.444 | 2.463 | 212 |
| 23.064 | 3.853 | 451 | 36.985 | 2.429 | 231 |
| 23.548 | 3.775 | 234 | 38.352 | 2.345 | 175 |
| 23.952 | 3.712 | 716 | 38.916 | 2.312 | 236 |
| 24.507 | 3.629 | 401 | | | |

**Table 3b. Powder X-Ray Diffraction Peak Data for Form 2**

| 2-theta° | d-spacing (A°) | Intensity | 2-theta° | d-spacing (A°) | Intensity |
|---|---|---|---|---|---|
| 5.116 | 17.259 | 8374 | 24.540 | 3.625 | 811 |
| 5.843 | 15.114 | 25293 | 25.131 | 3.541 | 261 |
| 7.209 | 12.253 | 721 | 25.878 | 3.440 | 1149 |
| 8.461 | 10.443 | 355 | 26.166 | 3.403 | 472 |
| 8.923 | 9.902 | 2772 | 26.998 | 3.300 | 391 |
| 9.750 | 9.064 | 10787 | 28.130 | 3.170 | 291 |
| 10.272 | 8.604 | 766 | 28.619 | 3.117 | 218 |
| 11.758 | 7.520 | 438 | 29.655 | 3.010 | 683 |
| 12.470 | 7.093 | 9909 | 29.928 | 2.983 | 1090 |
| 13.003 | 6.803 | 3449 | 30.848 | 2.896 | 670 |
| 13.955 | 6.341 | 326 | 31.174 | 2.867 | 305 |
| 14.826 | 5.970 | 465 | 31.702 | 2.820 | 338 |
| 15.407 | 5.746 | 7353 | 32.267 | 2.772 | 249 |
| 16.228 | 5.458 | 1002 | 33.025 | 2.710 | 240 |
| 16.945 | 5.228 | 39636 | 33.402 | 2.680 | 634 |
| 17.947 | 4.939 | 1863 | 34.302 | 2.612 | 218 |
| 18.136 | 4.888 | 2311 | 34.880 | 2.570 | 371 |
| 19.119 | 4.638 | 392 | 35.150 | 2.551 | 327 |
| 19.442 | 4.562 | 1240 | 36.009 | 2.492 | 252 |
| 21.056 | 4.216 | 1128 | 36.289 | 2.474 | 304 |
| 21.399 | 4.149 | 1181 | 37.017 | 2.427 | 359 |
| 21.783 | 4.077 | 2124 | 38.039 | 2.364 | 202 |
| 22.306 | 3.982 | 2048 | 38.394 | 2.343 | 289 |
| 22.984 | 3.866 | 375 | 39.039 | 2.305 | 380 |
| 23.609 | 3.765 | 781 | 39.740 | 2.266 | 347 |
| 23.981 | 3.708 | 2795 | | | |

## Claims

1. A Formula (rc1) compound, that is 3-methyl-3-(methylsulfonyl)-dihydrofuran-2(3H)-one 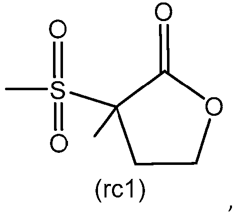 and stereoisomers thereof.

2. The compound of Claim 1 that is the Formula (R1) compound 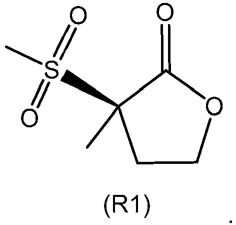 that is (R)-3-methyl-3-(methylsulfonyl)-dihydrofuran-2(3H)-one.

3. A process for preparing the Formula (rc1) compound of Claim 1, and stereoisomers thereof, according to the following rxn-1 procedure: 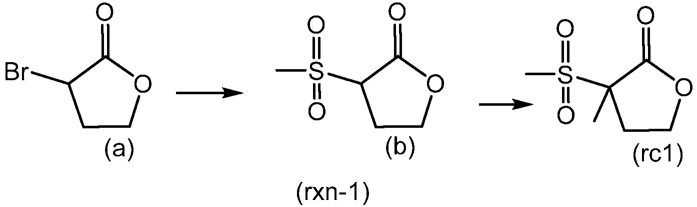 comprising:
a) reacting 3-bromotetrahydrofuran-2(3H)-one (a) with sodium methanesulfinate in the presence of solvent A, a phase transfer catalyst and optionally, a dehydrating agent, at a temperature of 40-100°C for 1-24 hours to prepare intermediate 3-(methylsulfonyl)-dihydrofuran-2(3H)-one (b);
b) cooling the mixture overnight at ambient temperature, filtering and washing the solids with solvent A;
c) reacting the solids with a base in the presence of a phase transfer catalyst, a methylating agent, solvent B, and optionally, a dehydrating agent, at a temperature of 40-100°C for a period of 4-48 hours;
d) charging the reactor with additional methylating agent and base while heating for an additional 4-hours;
e) filtering the solids while retaining the solvent filtrate;
f) washing the solids with solvent B while again retaining the solvent filtrate and then heating the combined filtrates to 65°C while distilling off all but 1.5 volumes;
g) exchanging the solvent with solvent A, cooling and seeding; and
h) cooling further to induce crystallization, filtering and washing the crystals with solvent A to afford the final product, 3-methyl-3-(methylsulfonyl)-dihydrofuran-2(3H)-one (rc1).

4. The process of Claim 3, further comprising a dehydrating agent, selected from the group consisting of sodium sulfate, calcium chloride and magnesium sulfate.

5. The process of Claim 3 or 4, wherein solvent A is methanol, ethanol or isopropanol, solvent B is acetone or n-butanol, the base is selected from the group consisting of sodium carbonate, potassium carbonate, cesium carbonate and tripotassium phosphate, the phase transfer catalyst is selected from methytrioctylammonium chloride, PEG 400 or PEG 300 and the methylating agent is selected from the group consisting of iodomethane, bromomethane, chloromethane, methyl tosylate, dimethyl sulfate, dimethyl carbonate and diazomethane.

6. The process of Claim 5, wherein solvent A is isopropanol, solvent B is acetone, the phase transfer catalyst is methytrioctylammonium chloride or PEG 400, the methylating agent is dimethyl sulfate and the base is sodium carbonate or potassium carbonate.

7. The process of Claim 6, comprising:
a) reacting 3-bromotetrahydrofuran-2(3H)-one with sodium methanesulfinate in the presence of isopropanol, methytrioctylammonium chloride and magnesium sulfate at a temperature of 80°C for 3 hours to prepare intermediate 3-(methylsulfonyl)dihydrofuran-2(3H)-one;
b) cooling the mixture overnight at ambient temperature, and isolating the solids by dissolution in isopropanol, filtering and washing the solids with isopropanol;
c) reacting the solids with potassium carbonate in the presence of methyltrioctylammonium chloride, dimethyl sulfate and magnesium sulfate in acetone at a temperature of 60°C for 7 hours;
d) charging the reactor with additional dimethyl sulfate and potassium carbonate while heating for an additional 4-hours;
e) filtering the solids while retaining the acetone filtrate;
f) washing the solids with acetone while again retaining the acetone filtrate and then heating the combined filtrates to 65°C while distilling off all but 1.5 volumes;
g) exchanging the acetone with isopropanol and cooling the mixture to 35°C and seeding; and
h) cooling further to 25°C to induce crystallization, filtering and washing the crystals with isopropanol to afford 3-methyl-3-(methylsulfonyl)-dihydrofuran-2(3H)-one (rc1).

8. A process for preparing a Formula (rc1) compound of Claim 1,
and stereoisomers thereof, according to the following rxn-t procedure: 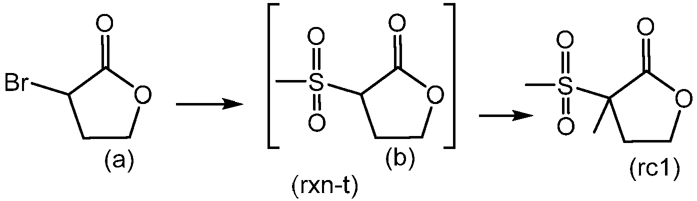 comprising:
a) reacting 3-bromodihydrofuran-2(3H)-one (a) with sodium methanesulfinate in the presence of a phase transfer catalyst, solvent B, and optionally, a dehydrating agent in a jacketed reactor for 7-11 hours at 50-80°C;
b) cooling the mixture to room temperature while stirring overnight for 12 hours; filtering the reaction mixture, retaining the filtrate, washing the solids with solvent B while retaining the filtrate;
c) reacting the combined filtrates with a methylating agent, a base and optionally, a dehydrating agent while slowly heating the reactants to 50-80°C for 15-20 hours;
d) adding more base and methylating agent to the mixture and heating for 3-6 more hours;
e) cooling the mixture to ambient temperature, filtering to remove the solids while retaining the filtrate;
f) washing the solids with solvent B, while again retaining the filtrate and heating the combined filtrates to 60-70°C; then distilling off all but 1.5 volumes; and
g) exchanging solvent B with solvent A while cooling to 30-40°C and seeding; then cooling further to 20-25°C to afford 3-methyl-3-(methylsulfonyl)-dihydrofuran-2(3H)-one (rc1).

9. The process of Claim 8, further comprising a dehydrating agent selected from the group consisting of sodium sulfate, calcium chloride and magnesium sulfate.

10. The process of Claim 8 or 9, wherein solvent B is acetone, the phase transfer catalyst is methytrioctylammonium chloride or PEG 400, the methylating agent is selected from the group consisting of iodomethane, bromomethane, chloromethane, methyl tosylate and dimethyl sulfate, the base is selected from the group consisting of sodium carbonate, potassium carbonate, cesium carbonate and tripotassium phosphate.

11. The process of Claim 8, comprising:
a) reacting 3-bromodihydrofuran-2(3H)-one with sodium methanesulfinate in the presence of a methyltrioctylammonium chloride, magnesium sulfate and acetone in a jacketed reactor for 9 hours to reflux at 56°C;
b) cooling the mixture to room temperature while stirring overnight for 12 hours; filtering the reaction mixture while retaining the filtrate and washing the solids with acetone again retaining the filtrate;
c) reacting the combined filtrates with dimethyl sulfate, anhydrous potassium carbonate and anhydrous magnesium sulfate while slowly heating the reactants to reflux at 56°C for 15 hours;
d) adding more potassium carbonate and dimethyl sulfate to the mixture and heating for 4 more hours;
e) cooling the mixture to ambient temperature and filtering to remove solids while retaining the filtrate;
f) washing the solids with acetone while again retaing the filtrate and heating the combined filtrates to 65°C; then distilling off all of the acetone but for 1.5 volumes; and
g) exchanging the acetone with isopropanol while cooling to 35°C and seeding; then cooling further to 20°C to afford 3-methyl-3-(methylsulfonyl)dihydrofuran-2(3H)-one.

12. A process for preparing 4-(4-chloro-2-oxopyridin-1(2H)-yl)-2-methyl-2-(methylsulfonyl)butanoic acid (rc2) from 3-methyl-3-(methylsulfonyl)-dihydrofuran-2(3H)-one (rc1), comprising:
a) dissolving potassium tert-butoxide in anhydrous DMSO and then adding 4-chloropyridin-2(1H)-one while maintaining the temperature between 18-27°C;
b) adding in a mixture of 3-methyl-3-(methylsulfonyl)-dihydrofuran-2(3H)-one (rc1) and benzyltriethylammonium bromide and heating to 85°C for 2 hours;
c) aging the reactants for 17-20 hours at 85°C;
d) adding isopropyl acetate and stirring for 1 hour at 60°C;
e) cooling to 20°C over an hour and aged for an hour; filtering the solids and washing with isopropyl acetate and drying overnight at 50°C to yield the potassium salt of 4-(4-chloro-2-oxopyridin-1(2H)-yl)-2-methyl-2-(methylsulfonyl)butanoic acid;
f) dissolving the dried solids in water and treating with methanol and methyl tetrahydrofuran and charging with concentrated HCl;
g) stirring the slurry at room temperature for 2 hours and cooling to 0-5°C while stirring for an additional hour; and
h) filtering the slurry and washing wth water:MeOH (8:2) and then drying in vacuo under nitrogen to afford 4-(4-chloro-2-oxopyridin-1(2H)-yl)-2-methyl-2-(methylsulfonyl)butanoic acid.

13. A process for preparing 4-(4-chloro-2-oxopyridin-1(2H)-yl)-2-methyl-2-(methylsulfonyl)butanoic acid (rc2) from 3-methyl-3-(methylsulfonyl)-dihydrofuran-2(3H)-one (rc1), comprising:
a) charging a reactor under nitrogen with PEG 400, 2-methyl-2-butanol and 4-chloro-2-hydroxypyridine;
b) adjusting the reactor temperature to 18-25°C and adding potassium tert-butoxide while stirring;
c) aging the reaction mixture for 30-60 minutes at 40-50°C;
d) adding 3-methyl-3-(methylsulfonyl)dihydrofuran-2(3H)-one (rc1) to the reactor and heating to 100°C over 15-30 minutes;
e) maintaining the temperature at 95-100°C for 12-24 hours
f) cooling the reactants to 50-60°C, diluting with water and adjusting the pH with HCl to 1-2;
g) cooling the reactants to 0-5°C over 1-3 hours, aging for 1-2 hours and filtering the resulting solids; and
h) washing the solids with water and drying to afford 4-(4-chloro-2-oxopyridin-1(2H)-yl)-2-methyl-2-(methylsulfonyl)butanoic acid (rc2).

14. A process for preparing (R)-4-(4-chloro-2-oxopyridin-1(2H)-yl)-2-methyl-2-(methylsulfonyl)-butanoic acid (R2) from (R)-3-methyl-3-(methylsulfonyl)-dihydrofuran-2(3H)-one (R1), comprising:
a) dissolving potassium tert-butoxide in anhydrous DMSO and then adding 4-chloropyridin-2(1H)-one while maintaining the temperture between 18-27°C;
b) adding in a mixture of (R)-3-methyl-3-(methylsulfonyl)dihydrofuran-2(3H)-one and benzyltriethylammonium bromide and heating to 85°C for 2 hours;
c) aging the reactants for 17-20 hours at 85°C;
d) adding isopropyl acetate and stirring for 1 hour at 60°C;
e) cooling to 20°C over an hour and aged for an hour; filtering the solids and washing with isopropyl acetate and drying overnight at 50°C to yield the potassium salt of (R)-4-(4-chloro-2-oxopyridin-1(2H)-yl)-2-methyl-2-(methylsulfonyl)-butanoic acid;
f) dissolving the dried solids in water and treating with methanol and methyl tetrahydrofuran and charging with concentrated HCl;
g) stirring the slurry at room temperature for 2 hours and cooling to 0-5°C while stirring for an additional hour; and
h) filtering the slurry and washing with water:MeOH (8:2) and then drying in vacuo under nitrogen to afford (R)-4-(4-chloro-2-oxopyridin-1(2H)-yl)-2-methyl-2-(methylsulfonyl)-butanoic acid.

## Patentansprüche

1. Verbindung der Formel (rc1), die 3-Methyl-3-(methylsulfonyl)-dihydrofuran-2(3H)-on ist 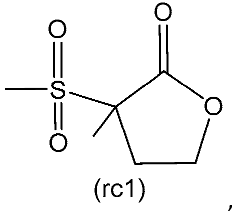 und deren Stereoisomere.

2. Verbindung nach Anspruch 1, die die Verbindung der Formel (R1) ist, 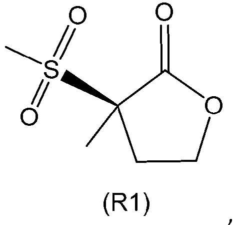 die (R)-3-Methyl-3-(methylsulfonyl)-dihydrofuran-2(3H)-on ist.

3. Verfahren zur Herstellung der Verbindung der Formel (rc1) nach Anspruch 1 und deren Stereoisomere gemäß dem folgenden Verfahren rxn-1: 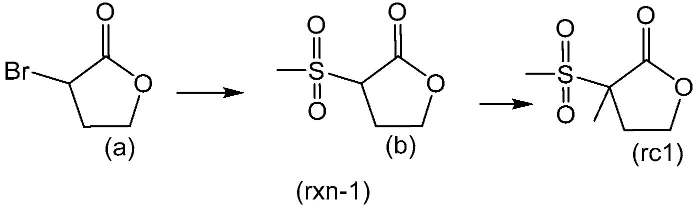 umfassend:
a) Reagieren von 3-Bromtetrahydrofuran-2(3H)-on (a) mit Natriummethansulfinat in Gegenwart von Lösungsmittel A, einem Phasentransferkatalysator und gegebenenfalls einem Dehydratisierungsmittel bei einer Temperatur von 40-100°C für 1-24 Stunden, um das Zwischenprodukt 3-(Methylsulfonyl)-dihydrofuran-2(3H)-on (b) herzustellen;
b) Abühlen der Mischung über Nacht bei Umgebungstemperatur, Filtrieren und Waschen der Feststoffe mit Lösungsmittel A;
c) Reagieren der Feststoffe mit einer Base in Gegenwart eines Phasentransferkatalysators, eines Methylierungsmittels, des Lösungsmittels B und gegebenenfalls eines Dehydratisierungsmittels bei einer Temperatur von 40-100°C für einen Zeitraum von 4-48 Stunden;
d) Befüllen des Reaktors mit zusätzlichem Methylierungsmittel und Base unter Erhitzen für weitere 4 Stunden;
e) Filtrieren der Feststoffe unter Zurückhalten des Lösungsmittelfiltrats;
f) Waschen der Feststoffe mit Lösungsmittel B unter erneutem Zurückhalten des Lösungsmittelfiltrats und anschließendes Erhitzen der vereinigten Filtrate auf 65°C unter Abdestillieren aller bis auf 1,5 Volumenanteile;
g) Austauschen des Lösungsmittels gegen Lösungsmittel A, Abkühlen und Beimpfen; und
h) weiteres Abkühlen, um die Kristallisation zu induzieren, Filtrieren und Waschen der Kristalle mit Lösungsmittel A, um das Endprodukt 3-Methyl-3-(methylsulfonyl)-dihydrofuran-2(3H)-on (rc1) zu erhalten.

4. Verfahren nach Anspruch 3, ferner umfassend ein Dehydratisierungsmittel, ausgewählt aus der Gruppe bestehend aus Natriumsulfat, Calciumchlorid und Magnesiumsulfat.

5. Verfahren nach Anspruch 3 oder 4, wobei Lösungsmittel A Methanol, Ethanol oder Isopropanol ist, Lösungsmittel B Aceton oder n-Butanol ist, die Base ausgewählt ist aus der Gruppe bestehend aus Natriumcarbonat, Kaliumcarbonat, Cäsiumcarbonat und Trikaliumphosphat, der Phasentransferkatalysator ausgewählt ist aus Methyltrioctylammoniumchlorid, PEG 400 oder PEG 300 und das Methylierungsmittel ausgewählt ist aus der Gruppe bestehend aus Jodmethan, Brommethan, Chlormethan, Methyltosylat, Dimethylsulfat, Dimethylcarbonat und Diazomethan.

6. Verfahren nach Anspruch 5, wobei das Lösungsmittel A Isopropanol ist, das Lösungsmittel B Aceton ist, der Phasentransferkatalysator Methyltrioctylammoniumchlorid oder PEG 400 ist, das Methylierungsmittel Dimethylsulfat ist und die Base Natriumcarbonat oder Kaliumcarbonat ist.

7. Verfahren nach Anspruch 6, umfassend:
a) Reagieren von 3-Bromtetrahydrofuran-2(3H)-on mit Natriummethansulfinat in Gegenwart von Isopropanol, Methyltrioctylammoniumchlorid und Magnesiumsulfat bei einer Temperatur von 80°C für 3 Stunden, um das Zwischenprodukt 3-(Methylsulfonyl)dihydrofuran-2(3H)-on herzustellen;
b) Abkühlen der Mischung über Nacht bei Umgebungstemperatur und Isolieren der Feststoffe durch Auflösen in Isopropanol, Filtrieren und Waschen der Feststoffe mit Isopropanol;
c) Reagieren der Feststoffe mit Kaliumcarbonat in Gegenwart von Methyltrioctylammoniumchlorid, Dimethylsulfat und Magnesiumsulfat in Aceton bei einer Temperatur von 60°C für 7 Stunden;
d) Befüllen des Reaktors mit zusätzlichem Dimethylsulfat und Kaliumcarbonat unter Erhitzen für weitere 4 Stunden;
e) Filtrieren der Feststoffe unter Zurückhalten des Acetonfiltrats;
f) Waschen der Feststoffe mit Aceton, wobei das Acetonfiltrat erneut zurückgehalten wird, und anschließendes Erhitzen der kombinierten Filtrate auf 65°C unter Abdestillation aller außer 1,5 Volumenanteile;
g) Austauschen des Acetons gegen Isopropanol und Abkühlen der Mischung auf 35°C und Beimpfen; und
h) weiteres Abkühlen auf 25°C, um die Kristallisation zu induzieren, Filtrieren und Waschen der Kristalle mit Isopropanol, um 3-Methyl-3-(methylsulfonyl)-dihydrofuran-2(3H)-on (rc1) zu erhalten.

8. Verfahren zur Herstellung einer Verbindung der Formel (rc1) gemäß Anspruch 1 und deren Stereoisomere gemäß dem folgenden Verfahren rxn-t, 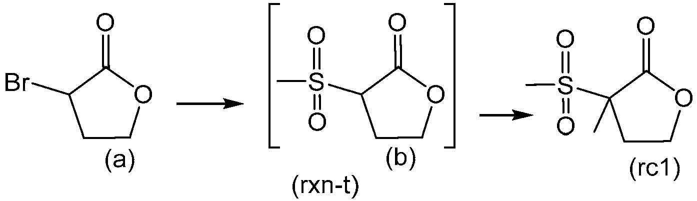 umfassend:
a) Reagieren von 3-Bromdihydrofuran-2(3H)-on (a) mit Natriummethansulfinat in Gegenwart eines Phasentransferkatalysators, des Lösungsmittels B und gegebenenfalls eines Dehydratisierungsmittels in einem Mantelreaktor für 7-11 Stunden bei 50-80°C;
b) Abkühlen der Mischung auf Raumtemperatur unter Rühren über Nacht für 12 Stunden; Filtrieren der Reaktionsmischung, Zurückhalten des Filtrats, Waschen der Feststoffe mit Lösungsmittel B unter Zurückhalten des Filtrats;
c) Reagieren der vereinigten Filtrate mit einem Methylierungsmittel, einer Base und gegebenenfalls einem Dehydratisierungsmittel, während die Reaktanten langsam auf 50-80°C für 15-20 Stunden erhitzt werden;
d) Zugabe von weiterer Base und Methylierungsmittel zu der Mischung und Erhitzen für weitere 3-6 Stunden;
e) Abkühlen der Mischung auf Umgebungstemperatur, Filtrieren, um die Feststoffe zu entfernen, unter Zurückhalten des Filtrats;
f) Waschen der Feststoffe mit Lösungsmittel B, wobei das Filtrat erneut zurückbehalten wird, und Erhitzen der vereinigten Filtrate auf 60-70°C; anschließendes Abdestillieren aller außer 1,5 Volumenanteilen; und
g) Austauschen des Lösungsmittels B durch Lösungsmittel A, während auf 30-40°C abgekühlt und beimpft wird; anschließendes weiteres Abkühlen auf 20-25°C, um 3-Methyl-3-(methylsulfonyl)-dihydrofuran-2(3H)-on (rc1) zu erhalten.

9. Verfahren nach Anspruch 8, ferner umfassend ein Dehydratisierungsmittel, ausgewählt aus der Gruppe bestehend aus Natriumsulfat, Calciumchlorid und Magnesiumsulfat.

10. Verfahren nach Anspruch 8 oder 9, wobei das Lösungsmittel B Aceton ist, der Phasentransferkatalysator Methyltrioctylammoniumchlorid oder PEG 400 ist, das Methylierungsmittel ausgewählt ist aus der Gruppe bestehend aus Jodmethan, Brommethan, Chlormethan, Methyltosylat und Dimethylsulfat, die Base ausgewählt ist aus der Gruppe bestehend aus Natriumcarbonat, Kaliumcarbonat, Cäsiumcarbonat und Trikaliumphosphat.

11. Verfahren nach Anspruch 8, umfassend:
a) Reagieren von 3-Bromdihydrofuran-2(3H)-on mit Natriummethansulfinat in Gegenwart von Methyltrioctylammoniumchlorid, Magnesiumsulfat und Aceton in einem doppelwandigen Reaktor für 9 Stunden unter Rückfluss bei 56°C;
b) Abkühlen der Mischung auf Raumtemperatur unter Rühren über Nacht für 12 Stunden; Filtrieren der Reaktionsmischung unter Zurückhalten des Filtrats und Waschen der Feststoffe mit Aceton unter erneutem Zurückhalten des Filtrats;
c) Reagieren der vereinigten Filtrate mit Dimethylsulfat, wasserfreiem Kaliumcarbonat und wasserfreiem Magnesiumsulfat unter langsamem Erhitzen der Reaktanten auf 56°C für 15 Stunden bis zum Rückfluss;
d) Zugabe von weiterem Kaliumcarbonat und Dimethylsulfat zur Mischung und Erhitzen für weitere 4 Stunden;
e) Abkühlen der Mischung auf Umgebungstemperatur und Filtrieren, um Feststoffe zu entfernen, unter Zurückhalten des Filtrats;
f) Waschen der Feststoffe mit Aceton, unter erneutem Zurückhalten des Filtrats, und Erhitzen der vereinigten Filtrate auf 65°C; anschließendes Abdestillieren des gesamten Acetons bis auf 1,5 Volumenanteile; und
g) Austauschen des Acetons gegen Isopropanol unter Abkühlen auf 35°C und Beimpfen; anschließendes weiteres Abkühlen auf 20°C, um 3-Methyl-3-(methylsulfonyl)dihydrofuran-2(3H)-on zu erhalten.

12. Verfahren zur Herstellung von 4-(4-Chlor-2-oxopyridin-1(2H)-yl)-2-methyl-2-(methylsulfonyl)butansäure (rc2) aus 3-Methyl-3-(methylsulfonyl)-dihydrofuran-2(3H)-on (rc1), umfassend:
a) Auflösen von Kalium-tert-butoxid in wasserfreiem DMSO und anschließendes Hinzufügen von 4-Chlorpyridin-2(1H)-on unter Beibehaltung einer Temperatur zwischen 18 und 27°C;
b) Zugabe einer Mischung aus 3-Methyl-3-(methylsulfonyl)-dihydrofuran-2(3H)-on (rc1) und Benzyltriethylammoniumbromid und Erhitzen auf 85°C für 2 Stunden;
c) Reifenlassen der Reaktanten für 17-20 Stunden bei 85°C;
d) Zugabe von Isopropylacetat und Rühren für 1 Stunde bei 60°C;
e) Abkühlen auf 20°C über einen Zeitraum von einer Stunde und Reifenlassen für eine Stunde; Filtrieren der Feststoffe und Waschen mit Isopropylacetat und Trocknen über Nacht bei 50°C, um das Kaliumsalz von 4-(4-Chlor-2-oxopyridin-1(2H)-yl)-2-methyl-2-(methylsulfonyl)butansäure zu erhalten;
f) Auflösen der getrockneten Feststoffe in Wasser und Behandeln mit Methanol und Methyltetrahydrofuran und Zugabe von konzentrierter HCl;
g) Rühren der Aufschlämmung bei Raumtemperatur für 2 Stunden und Abkühlen auf 0-5°C unter weiterem Rühren für eine weitere Stunde; und
h) Filtrieren der Aufschlämmung und Waschen mit Wasser:MeOH (8:2) und anschließendes Trocknen unter Vakuum unter Stickstoff, um 4-(4-Chlor-2-oxopyridin-1(2H)-yl)-2-methyl-2-(methylsulfonyl)butansäure zu erhalten.

13. Verfahren zur Herstellung von 4-(4-Chlor-2-oxopyridin-1(2H)-yl)-2-methyl-2-(methylsulfonyl)butansäure (rc2) aus 3-Methyl-3-(methylsulfonyl)-dihydrofuran-2(3H)-on (rc1), umfassend:
a) Befüllen eines Reaktors unter Stickstoff mit PEG 400, 2-Methyl-2-butanol und 4-Chlor-2-hydroxypyridin;
b) Einstellen der Reaktortemperatur auf 18-25°C und Zugabe von Kalium-tert-butoxid unter Rühren;
c) Reifenlassen des Reaktionsgemisches für 30-60 Minuten bei 40-50°C;
d) Zugabe von 3-Methyl-3-(methylsulfonyl)dihydrofuran-2(3H)-on (rc1) in den Reaktor und Erhitzen auf 100°C für 15-30 Minuten;
e) Halten der Temperatur für 12-24 Stunden bei 95-100°C;
f) Abkühlen der Reaktionsprodukte auf 50-60°C, Verdünnen mit Wasser und Einstellen des pH-Werts mit HCl auf 1-2;
g) Abkühlen der Reaktanten auf 0-5°C für 1-3 Stunden, Reifenlassen für 1-2 Stunden und Filtrieren der resultierenden Feststoffe; und
h) Waschen der Feststoffe mit Wasser und Trocknen, um 4-(4-Chlor-2-oxopyridin-1(2H)-yl)-2-methyl-2-(methylsulfonyl)butansäure (rc2) zu erhalten.

14. Verfahren zur Herstellung von (R)-4-(4-Chlor-2-oxopyridin-1(2H)-yl)-2-methyl-2-(methylsulfonyl)-butansäure (R2) aus (R)-3-Methyl-3-(methylsulfonyl)-dihydrofuran-2(3H)-on (R1), umfassend:
a) Auflösen von Kalium-tert-butoxid in wasserfreiem DMSO und anschließendes Hinzufügen von 4-Chlorpyridin-2(1H)-on unter Aufrechterhaltung einer Temperatur zwischen 18 und 27°C;
b) Zugabe einer Mischung aus (R)-3-Methyl-3-(methylsulfonyl)dihydrofuran-2(3H)-on und Benzyltriethylammoniumbromid und Erhitzen auf 85°C für 2 Stunden;
c) Reifenlassen der Reaktanten für 17-20 Stunden bei 85°C;
d) Zugabe von Isopropylacetat und Rühren bei 60°C für 1 Stunde;
e) Abkühlen auf 20°C für eine Stunde und Reifenlassen für eine Stunde; Filtrieren der Feststoffe und Waschen mit Isopropylacetat und Trocknen bei 50°C über Nacht, um das Kaliumsalz von (R)-4-(4-Chlor-2-oxopyridin-1(2H)-yl)-2-methyl-2-(methylsulfonyl)-butansäure zu erhalten;
f) Auflösen der getrockneten Feststoffe in Wasser und Behandeln mit Methanol und Methyltetrahydrofuran und Zugabe von konzentrierter HCl;
g) Rühren der Aufschlämmung bei Raumtemperatur für 2 Stunden und Abkühlen auf 0-5°C unter weiterem Rühren für eine weitere Stunde; und
h) Filtrieren der Aufschlämmung und Waschen mit Wasser:MeOH (8:2) und anschließendes Trocknen unter Vakuum unter Stickstoff, um (R)-4-(4-Chlor-2-oxopyridin-1(2H)-yl)-2-methyl-2-(methylsulfonyl)-butansäure zu erhalten.

## Revendications

1. Composé de formule (rc1), qui est la 3-méthyl-3-(méthylsulfonyl)-dihydrofuran-2(3H)-one 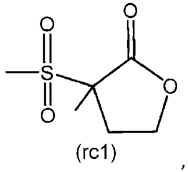 et ses stéréoisomères.

2. Composé selon la revendication 1 qui est le composé de formule (R1) 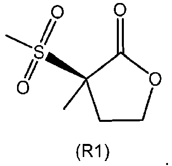 qui est la (R)-3-méthyl-3-(méthylsulfonyl)-dihydrofuran-2(3H)-one.

3. Procédé de préparation du composé de formule (rc1) de la revendication 1, et de ses stéréoisomères, selon la procédure rxn-1 suivante : 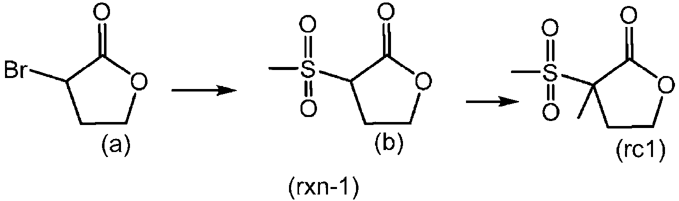 comprenant :
a) la réaction de 3-bromotétrahydrofuran-2(3H)-one (a) avec du méthanesulfinate de sodium en présence d'un solvant A, d'un catalyseur de transfert de phase et, optionnellement, d'un agent déshydratant, à une température de 40 à 100 °C pendant 1 à 24 heures pour préparer la 3-(méthylsulfonyl)-dihydrofuran-2(3H)-one (b) intermédiaire ;
b) le refroidissement du mélange toute une nuit à température ambiante, la filtration et le lavage des solides avec le solvant A ;
c) la réaction des solides avec une base en présence d'un catalyseur de transfert de phase, d'un agent de méthylation, d'un solvant B et, optionnellement, d'un agent déshydratant, à une température de 40 à 100 °C pendant une période de 4 à 48 heures ;
d) le chargement du réacteur avec un agent de méthylation et une base supplémentaires tout en chauffant pendant 4 heures supplémentaires ;
e) la filtration des solides tout en retenant le filtrat de solvant ;
f) le lavage des solides avec le solvant B tout en retenant à nouveau le filtrat de solvant, puis le chauffage des filtrats combinés à 65 °C tout en distillant tous les volumes sauf 1,5 ;
g) l'échange du solvant avec le solvant A, le refroidissement et l'ensemencement ; et
h) le refroidissement supplémentaire pour induire une cristallisation, la filtration et le lavage des cristaux avec le solvant A pour obtenir le produit final, la 3-méthyl-3-(méthylsulfonyl)-dihydrofuran-2(3H)-one (rc1).

4. Procédé selon la revendication 3, comprenant en outre un agent déshydratant, choisi dans le groupe consistant en le sulfate de sodium, le chlorure de calcium et le sulfate de magnésium.

5. Procédé selon la revendication 3 ou 4, dans lequel le solvant A est le méthanol, l'éthanol ou l'isopropanol, le solvant B est l'acétone ou le n-butanol, la base est choisie dans le groupe consistant en le carbonate de sodium, le carbonate de potassium, le carbonate de césium et le phosphate tripotassique, le catalyseur de transfert de phase est choisi parmi le chlorure de méthyltrioctylammonium, le PEG 400 ou le PEG 300 et l'agent de méthylation est choisi dans le groupe consistant en l'iodométhane, le bromométhane, le chlorométhane, le tosylate de méthyle, le sulfate de diméthyle, le carbonate de diméthyle et le diazométhane.

6. Procédé selon la revendication 5, dans lequel le solvant A est l'isopropanol, le solvant B est l'acétone, le catalyseur de transfert de phase est le chlorure de méthyltrioctylammonium ou le PEG 400, l'agent de méthylation est le sulfate de diméthyle et la base est le carbonate de sodium ou le carbonate de potassium.

7. Procédé selon la revendication 6, comprenant :
a) la réaction de 3-bromotétrahydrofuran-2(3H)-one avec du méthanesulfinate de sodium en présence d'isopropanol, de chlorure de méthyltrioctylammonium et de sulfate de magnésium à une température de 80 °C pendant 3 heures pour préparer la 3-(méthylsulfonyl)dihydrofuran-2(3H)-one intermédiaire ;
b) le refroidissement du mélange toute une nuit à température ambiante, et l'isolement des solides par dissolution dans de l'isopropanol, filtration et lavage des solides avec de l'isopropanol ;
c) la réaction des solides avec du carbonate de potassium en présence de chlorure de méthyltrioctylammonium, de sulfate de diméthyle et de sulfate de magnésium dans de l'acétone à une température de 60 °C pendant 7 heures ;
d) le chargement du réacteur avec du sulfate de diméthyle et du carbonate de potassium supplémentaires tout en chauffant pendant 4 heures supplémentaires ;
e) la filtration des solides tout en retenant le filtrat d'acétone ;
f) le lavage des solides avec de l'acétone tout en retenant à nouveau le filtrat d'acétone puis le chauffage des filtrats combinés à 65 °C tout en distillant tous les volumes sauf 1,5 ;
g) l'échange de l'acétone avec de l'isopropanol et le refroidissement du mélange à 35 °C et l'ensemencement ; et
h) le refroidissement supplémentaire à 25 °C pour induire une cristallisation, la filtration et le lavage des cristaux avec de l'isopropanol pour obtenir de la 3-méthyl-3-(méthylsulfonyl)-dihydrofuran-2(3H)-one (rc1).

8. Procédé de préparation d'un composé de formule (rc1) de la revendication 1, et de ses stéréoisomères, selon la procédure rxn-t suivante : 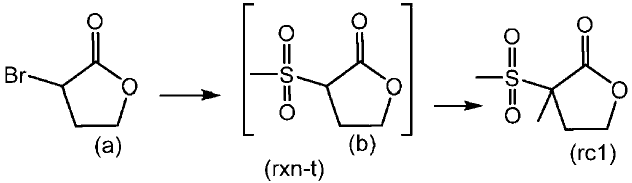 comprenant :
a) la réaction de 3-bromodihydrofuran-2(3H)-one (a) avec du méthanesulfinate de sodium en présence d'un catalyseur de transfert de phase, du solvant B, et optionnellement, d'un agent déshydratant dans un réacteur chemisé pendant 7 à 11 heures à 50 à 80 °C ;
b) le refroidissement du mélange à température ambiante tout en agitant toute une nuit pendant 12 heures ; la filtration du mélange de réaction, la retenue du filtrat, le lavage des solides avec le solvant B tout en retenant le filtrat ;
c) la réaction des filtrats combinés avec un agent de méthylation, une base et, optionnellement, un agent déshydratant tout en chauffant lentement les réactifs à 50 à 80 °C pendant 15 à 20 heures ;
d) l'ajout de plus de base et d'agent de méthylation au mélange et le chauffage pendant 3 à 6 heures de plus ;
e) le refroidissement du mélange à température ambiante, la filtration pour éliminer les solides tout en retenant le filtrat ;
f) le lavage des solides avec le solvant B, tout en retenant à nouveau le filtrat et en chauffant les filtrats combinés à 60 à 70 °C ; puis la distillation de tous les volumes sauf 1,5 ; et
g) l'échange du solvant B avec le solvant A pendant le refroidissement à 30 à 40 °C et l'ensemencement ; puis le refroidissement supplémentaire à 20 à 25 °C pour obtenir de la 3-méthyl-3-(méthylsulfonyl)-dihydrofuran-2(3H)-one (rc1).

9. Procédé selon la revendication 8, comprenant en outre un agent déshydratant choisi dans le groupe consistant en le sulfate de sodium, le chlorure de calcium et le sulfate de magnésium.

10. Procédé selon la revendication 8 ou 9, dans lequel le solvant B est l'acétone, le catalyseur de transfert de phase est le chlorure de méthyltrioctylammonium ou le PEG 400, l'agent de méthylation est choisi dans le groupe consistant en l'iodométhane, le bromométhane, le chlorométhane, le tosylate de méthyle et le sulfate de diméthyle, la base est choisie dans le groupe consistant en le carbonate de sodium, le carbonate de potassium, le carbonate de césium et le phosphate tripotassique.

11. Procédé selon la revendication 8, comprenant :
a) la réaction de 3-bromodihydrofuran-2(3H)-one avec du méthanesulfinate de sodium en présence d'un chlorure de méthyltrioctylammonium, de sulfate de magnésium et d'acétone dans un réacteur chemisé pendant 9 heures jusqu'au reflux à 56 °C ;
b) le refroidissement du mélange à température ambiante tout en agitant tout une nuit pendant 12 heures ; la filtration du mélange de réaction tout en retenant le filtrat et le lavage à nouveau des solides avec de l'acétone tout en retenant le filtrat ;
c) la réaction des filtrats combinés avec du sulfate de diméthyle, du carbonate de potassium anhydre et du sulfate de magnésium anhydre tout en chauffant lentement les réactifs au reflux à 56 °C pendant 15 heures ;
d) l'ajout de plus de carbonate de potassium et de sulfate de diméthyle au mélange et le chauffage pendant 4 heures de plus ;
e) le refroidissement du mélange à température ambiante et la filtration pour éliminer les solides tout en retenant le filtrat ;
f) le lavage des solides avec de l'acétone tout en retenant à nouveau le filtrat et le chauffage des filtrats combinés à 65 °C ; puis la distillation de toute l'acétone sauf 1,5 volume ; et
g) l'échange de l'acétone avec de l'isopropanol pendant le refroidissement à 35 °C et l'ensemencement ; puis le refroidissement supplémentaire à 20 °C pour obtenir de la 3-méthyl-3-(méthylsulfonyl)dihydrofuran-2(3H)-one.

12. Procédé de préparation d'acide 4-(4-chloro-2-oxopyridin-1(2H)-yl)-2-méthyl-2-(méthylsulfonyl)butanoïque (rc2) à partir de 3-méthyl-3-(méthylsulfonyl)-dihydrofuran-2(3H)-one (rc1), comprenant :
a) la dissolution de tert-butoxyde de potassium dans du DMSO anhydre puis l'ajout de 4-chloropyridin-2(1H)-one tout en maintenant la température entre 18 et 27 °C ;
b) l'ajout dans un mélange de 3-méthyl-3-(méthylsulfonyl)-dihydrofuran-2(3H)-one (rc1) et de bromure de benzyltriéthylammonium et le chauffage à 85 °C pendant 2 heures ;
c) le vieillissement des réactifs pendant 17 à 20 heures à 85 °C ;
d) l'ajout d'acétate d'isopropyle et l'agitation pendant 1 heure à 60 °C ;
e) le refroidissement à 20 °C durant une heure et le vieillissement pendant une heure ; la filtration des solides et le lavage avec de l'acétate d'isopropyle et le séchage tout une nuit à 50 °C pour produire le sel de potassium d'acide 4-(4-chloro-2-oxopyridin-1(2H)-yl)-2-méthyl-2-(méthylsulfonyl)butanoïque ;
f) la dissolution des solides séchés dans l'eau et le traitement avec du méthanol et du méthyl tétrahydrofurane et le chargement avec du HCl concentré ;
g) l'agitation de la barbotine à température ambiante pendant 2 heures et le refroidissement à 0 à 5 °C tout en agitant pendant une heure supplémentaire ; et
h) la filtration de la barbotine et le lavage avec de l'eau:MeOH (8:2) puis le séchage sous vide sous azote pour obtenir de l'acide 4-(4-chloro-2-oxopyridin-1(2H)-yl)-2-méthyl-2-(méthylsulfonyl)butanoïque.

13. Procédé de préparation d'acide 4-(4-chloro-2-oxopyridin-1(2H)-yl)-2-méthyl-2-(méthylsulfonyl)butanoïque (rc2) à partir de 3-méthyl-3-(méthylsulfonyl)-dihydrofuran-2(3H)-one (rc1), comprenant :
a) le chargement d'un réacteur sous azote avec du PEG 400, du 2-méthyl-2-butanol et de la 4-chloro-2-hydroxypyridine ;
b) l'ajustement de la température du réacteur à 18 à 25 °C et l'ajout de tert-butoxyde de potassium tout en agitant ;
c) le vieillissement du mélange de réaction pendant 30 à 60 minutes à 40 à 50 °C ;
d) l'ajout de 3-méthyl-3-(méthylsulfonyl)dihydrofuran-2(3H)-one (rc1) au réacteur et le chauffage à 100 °C durant 15 à 30 minutes ;
e) le maintien de la température à 95 à 100 °C pendant 12 à 24 heures
f) le refroidissement des réactifs à 50 à 60 °C, la dilution avec de l'eau et la correction du pH avec du HCl à 1 à 2 ;
g) le refroidissement des réactifs à 0 à 5 °C durant 1 à 3 heures, le vieillissement pendant 1 à 2 heures et la filtration des solides résultants ; et
h) le lavage des solides avec de l'eau et le séchage pour obtenir de l'acide 4-(4-chloro-2-oxopyridin-1(2H)-yl)-2-méthyl-2-(méthylsulfonyl)butanoïque (rc2).

14. Procédé de préparation d'acide (R)-4-(4-chloro-2-oxopyridin-1(2H)-yl)-2-méthyl-2-(méthylsulfonyl)-butanoïque (R2) à partir de (R)-3-méthyl-3-(méthylsulfonyl)-dihydrofuran-2(3H)-one (R1), comprenant :
a) la dissolution de tert-butoxyde de potassium dans du DMSO anhydre puis l'ajout de 4-chloropyridin-2(1H)-one tout en maintenant la température entre 18 et 27 °C ;
b) l'ajout dans un mélange de (R)-3-méthyl-3-(méthylsulfonyl)dihydrofuran-2(3H)-one et de bromure de benzyltriéthylammonium et le chauffage à 85 °C pendant 2 heures ;
c) le vieillissement des réactifs pendant 17 à 20 heures à 85 °C ;
d) l'ajout d'acétate d'isopropyle et l'agitation pendant 1 heure à 60 °C ;
e) le refroidissement à 20 °C durant une heure et le vieillissement pendant une heure ; la filtration des solides et le lavage avec de l'acétate d'isopropyle et le séchage tout une nuit à 50 °C pour produire le sel de potassium d'acide (R)-4-(4-chloro-2-oxopyridin-1(2H)-yl)-2-méthyl-2-(méthylsulfonyl)-butanoïque ;
f) la dissolution des solides séchés dans l'eau et le traitement avec du méthanol et du méthyl tétrahydrofurane et le chargement avec du HCl concentré ;
g) l'agitation de la barbotine à température ambiante pendant 2 heures et le refroidissement à 0 à 5 °C tout en agitant pendant une heure supplémentaire ; et
h) la filtration de la barbotine et le lavage avec de l'eau:MeOH (8:2) puis le séchage sous vide sous azote pour obtenir de l'acide (R)-4-(4-chloro-2-oxopyridin-1(2H)-yl)-2-méthyl-2-(méthylsulfonyl)-butanoïque.
